# EUROPÄISCHE PATENTSCHRIFT

(11) **EP 1 669 028 B1**
(45) Veröffentlichungstag und Bekanntmachung des Hinweises auf die Patenterteilung: **09.01.2008**
(21) Anmeldenummer: 05025309.5
(22) Anmeldetag: 19.11.2005
(51) Int. Cl.: A61B 5/15

(54) **Lanzettenvorrichtung zum Erzeugen einer Einstichwunde und Lanzettenantriebs-Baugruppe**
Lancet device for forming an incision and lancet drive sub-assembly
Dispositif lancette pour formation d'une incision et sous-ensemble pour un actioneur d'une lancette

(30) Priorität: 10.12.2004 DE 102004059491
(43) Veröffentlichungstag der Anmeldung: 14.06.2006
(73) Patentinhaber: Roche Diagnostics GmbH, 68305 Mannheim (DE); F.HOFFMANN-LA ROCHE AG, 4070 Basel (CH)
(72) Erfinder: List, Hans, 64754 Hesseneck-Kailbach (DE)
(74) Vertreter: Pfeifer, Hans-Peter

(56) Entgegenhaltungen:
- EP-A- 1 384 438
- US-B1- 6 419 661

## Beschreibung

Die Erfindung betrifft eine Lanzettenvorrichtung zum Erzeugen einer Einstichwunde, insbesondere zum Entnehmen einer Körperflüssigkeit für Diagnosezwecke, und eine Lanzettenantriebs-Baugruppe für eine Lanzettenvorrichtung.

Um für analytisch-diagnostische Zwecke eine geringe Menge Körperflüssigkeit (Blut und/oder interstitielle Flüssigkeit) aus einem Körperteil (meist aus einem Finger oder Ohrläppchen) zu entnehmen, werden Lanzetten verwendet, die zur Erzeugung einer Einstichwunde in das entsprechende Körperteil gestochen werden. Soweit dies manuell geschieht, ist speziell trainiertes Personal erforderlich. Dennoch ist der Einstich mit erheblichem Schmerz verbunden.

Bereits seit Langem werden Blutentnahmesysteme verwendet, die aus einer Lanzettenvorrichtung und zugehörigen, für die jeweilige Lanzettenvorrichtung speziell angepaßten Lanzetten bestehen. In einem Gehäuse der Lanzettenvorrichtung befindet sich ein Lanzettenantrieb, durch den eine Lanzette mechanisch in die Haut gestochen wird. Als Antriebselement für die Einstichbewegung dient eine Feder. Zu Beginn der Entwicklung waren sehr einfache Konstruktionen gebräuchlich, bei denen die Lanzette unmittelbar an einem Ende einer in einem länglichen Gehäuse angeordneten Druckfeder befestigt war (z.B. US-Patent 4,469,110).

Derartige Blutentnahmesysteme wurden jedoch den hohen Anforderungen nicht gerecht, die zu erfüllen sind, wenn eine regelmäßige Überwachung analytischer Werte des Blutes erforderlich ist. Dies gilt insbesondere für Diabetiker, die ihren Blutzuckerspiegel häufig kontrollieren sollten, um ihn durch Insulininjektionen innerhalb bestimmter Sollgrenzen halten zu können. Durch umfangreiche wissenschaftliche Untersuchungen wurde bewiesen, daß mittels einer Intensivtherapie mit mindestens vier Blutanalysen pro Tag eine dramatische Reduzierung schwerster Spätschäden des Diabetis Mellitus (beispielsweise eine Retinopathie mit resultierender Erblindung des Patienten) erreicht werden kann.

Diese Intensivtherapie setzt voraus, daß die Blutentnahme mit einem möglichst geringen Schmerz verbunden ist. Mit dem Ziel, diesbezüglich eine Verbesserung zu erreichen, wurden zahlreiche unterschiedliche Blutentnahmesysteme entwickelt.

Eine schmerzarme Blutentnahme ermöglichen Lanzettenvorrichtungen, deren Lanzettenantrieb einen Antriebsrotor einschließt, auf den einerseits (antriebsseitig) die Antriebsfeder derartig wirkt, daß er von dieser in eine Drehbewegung um eine Rotationsachse versetzt werden kann, und der andererseits über einen (abtriebsseitigen) Kopplungsmechanismus derartig mit der Lanzette gekoppelt ist, daß die aus der Entspannungsbewegung der Antriebsfeder resultierende Rotation des Antriebsrotors in eine Einstichbewegung umgesetzt wird. Die vorliegende Erfindung bezieht sich auf eine Lanzettenvorrichtung mit einem solchen Rotor-Lanzettenantrieb, der in verschiedenen Ausführungsformen bekannt ist.

Der abtriebsseitige Kopplungsmechanismus ist in der Regel so konstruiert, daß die Lanzette während der gesamten Einstich- und Rückführbewegung (Antriebsphase der Bewegung des Lanzettenantriebs) mit dem Antriebsrotor gekoppelt ist und dadurch die Lanzettenbewegung vollständig durch die entsprechende Bewegung des Antriebsrotors kontrolliert wird. Ein frühes Beispiel einer derartigen Konstruktion zeigt das US-Patent 4924879. Modernere Ausführungsformen sind beispielsweise in den US-Patenten 6409740 und 6419661 beschrieben.

Das Spannen der bekannten Rotor-Lanzettenantriebe erfolgt in der Regel dadurch, daß der Rotor mittels eines geeigneten "antriebsseitigen Kopplungsmechanismus" gegen die Kraft der Antriebsfeder zurückgedreht wird. In neuerer Zeit ist eine Konstruktion bekannt geworden, bei der zum Spannen der Antriebsfeder ein Spannrotor verwendet wird. Dadurch läßt sich erreichen, daß das Spannen des Lanzettenantriebs in einer Spannphase seiner Bewegung durch eine Drehung des Spannrotors erfolgt, die mit der Drehung des Antriebsrotors in der Antriebsphase gleichgerichtet ist. Dieses Antriebsprinzip wird auch als OWADAC (one way alternating drive and cocking) bezeichnet. Ein solcher Rotorantrieb ist beispielsweise in der EP 1384438 A1 beschrieben.

Trotz umfangreicher Entwicklungsarbeiten besteht weiter ein großes Interesse an einer Lanzettenvorrichtung, welche die schwierigen und teilweise gegenläufigen Anforderungen (minimales Schmerzempfinden, einfache Bedienbarkeit, kompakte Bauweise, kostengünstige Konstruktion) gleichzeitig möglichst weitgehend erfüllt. Als weitere Anforderung tritt hinzu, daß die Lanzettenvorrichtung beim Gebrauch nicht oder möglichst wenig durch austretendes Blut verschmutzt wird. Aufgabe der Erfindung ist es, diese Anforderungen möglichst weitgehend zu erfüllen.

Diese Aufgabe wird gelöst durch eine Lanzettenvorrichtung zum Erzeugen einer Einstichwunde in einer Hautoberfläche, insbesondere zum Entnehmen einer Körperflüssigkeit für Diagnosezwecke, umfassend ein Gehäuse, in dem eine Lanzette auf einem Einstichweg beweglich ist, einen Lanzettenantrieb mit einem durch eine Antriebsfeder antreibbaren Antriebsrotor, einen Lanzetten-Kopplungsmechanismus, durch den während eines Arbeitszyklus des Lanzettenantriebs eine Drehbewegung des Antriebsrotors in eine Einstich- und Rückführbewegung der Lanzette umgesetzt wird, ein relativ zu der Lanzette und relativ zu dem Gehäuse bewegliches Referenzelement, das sich zum Zeitpunkt des Einstichs in einer definierten Position relativ zu dem Lanzettenantrieb (und damit zu dem Einstichweg) befindet und mit einer Kontaktfläche an der Hautoberfläche anliegt, so daß durch die Einstichbewegung eine Einstichwunde mit reproduzierbarer Einstichtiefe erzeugt wird, und einen Referenzelement-Kopplungsmechanismus, der zum Bewegen des Referenzelementes mit dem Lanzettenantrieb gekoppelt ist.

Um für Diagnosezwecke mit einem möglichst schmerzarmen Einstich eine ausreichende Menge Blut zu gewinnen, ist die optimale Einstichtiefe von zentraler Bedeutung. Variationen von 0,05 mm können eine signifikante Änderung des Schmerzempfindens und/oder der bei einem Einstich gewonnenen Menge Blut zur Folge haben.

Bei Lanzettenvorrichtungen mit Rotor-Lanzettenantrieb wird die Bewegung der Lanzette, wie beschrieben, durch die Kopplung mit dem Antriebsrotor präzise kontrolliert. Die Einstichtiefe entspricht dabei dem Abstand zwischen dem tiefsten Punkt (Umkehrpunkt) der Lanzettenbewegung und der Ebene einer Hautkontaktfläche, die bei bekannten Konstruktionen als ringförmige Andruckfläche eine Öffnung am vorderen Ende des Gehäuses umgibt. Die Andruckfläche wird gegen das Körperteil gedrückt, aus dem Blut entnommen werden soll. Zur Einstellung der Einstichtiefe ist ihre Axialposition (d.h. die Position in Einstichrichtung) verstellbar.

Ist die von der Andruckfläche umgebene Öffnung nur wenig größer als der Lanzettendurchmesser, so ist zwar die Einstichtiefe gut reproduzierbar, jedoch ist die durch einen Einstich gewonnene Blutmenge relativ gering und insbesondere für integrierte Systeme, bei denen eine Blutprobe in der Lanzettenvorrichtung ausgewertet werden soll, nicht ausreichend. Ist die von der Andruckfläche umgebene Gehäuseöffnung größer, so daß sich die Haut in sie hineinwölbt, läßt sich wegen einer erhöhten Durchblutung mit einem Einstich eine größere Menge Blut gewinnen.

Im Rahmen der Erfindung wurde festgestellt, daß mit einer stationären Andruckfläche am vorderen Ende des Gehäuses vielfach keine optimale Reproduzierbarkeit der Einstichtiefe zu erreichen ist, insbesondere wenn sich die Haut beim Anpressen einer Lanzettenvorrichtung in die von der Andruckfläche umgebene Gehäuseöffnung hineinwölbt. Deren Innenrand stellt dann nur eine ungenaue Referenz für die Einstichtiefe dar. Bei kleineren Gehäuseöffnungen ist dieser Effekt weniger stark ausgeprägt, jedoch wird dann weniger Blut gewonnen, und es besteht ein erhöhtes Risiko, daß die Lanzettenvorrichtung durch aus der Einstichwunde austretendes Blut verschmutzt wird.

Bisher wurde versucht, zur Lösung dieses Problems einen möglichst optimalen Kompromiß für den Innendurchmesser der Andruckfläche, d.h. die Größe der Gehäuseöffnung, zu finden. Abweichend von dieser üblichen Vorgehensweise weist eine erfindungsgemäße Lanzettenvorrichturig ein (relativ zu der Lanzette und dem Gehäuse) bewegliches Referenzelement auf, das während des Einstichs mit einer Kontaktfläche an der Haut eines Patienten anliegt. Zur Bewegung des Referenzelementes dient ein Referenzelement-Kopplungsmechanismus, der zusätzlich zu dem Lanzetten-Kopplungsmechanismus vorhanden ist. Das bewegliche Referenzelement befindet sich während des Einstichs, zumindest zum Zeitpunkt des maximalen Eindringens der Lanzettenspitze in die Haut, in einer definierten Position relativ zu dem Lanzettenantrieb. Anhand der nachfolgenden Beschreibung wird deutlich, daß mit einer solchen Konstruktion eine sehr gute Reproduzierbarkeit der Tiefe der Einstichwunde trotz Wölbung der Haut erzielt werden kann.

Das Referenzelement einer erfindungsgemäßen Lanzettenvorrichtung kann als Probenaufnahmeeinheit ausgebildet sein, die nebeneinander eine Führung für die Lanzette und einen Probenaufnahmekanal zum Aufnehmen einer Blutprobe aufweist. Von der Probenaufnahmeeinheit kann die Blutprobe zu einer weiteren Bearbeitungsstation (beispielsweise um sie zu analysieren) weitergeleitet werden. Bevorzugt ist jedoch eine Reaktionszone mit Reagenzien integraler Bestandteil der Probeaufnahmeeinheit, so daß die Probeaufnahmeeinheit ein vollständiges Analyseelement bildet. Die Reaktion der Probe mit den Reagenzien in der Reaktionszone führt zu einer Änderung eines physikalischen Zustandes (beispielsweise zu einer Farbänderung oder bei elektrochemischen Analyseelementen zur Änderung eines Stromflusses) die als Grundlage für die Ermittlungen der Konzentration eines in der Probe enthaltenen Analyten verwendet werden kann. Da solche Analyseelemente bekannt sind, ist eine nähere Erläuterung der Testprinzipien nicht erforderlich. Die Analyseelemente (oder sonstige Probeaufnahmeeinheiten) werden nach einmaliger Verwendung entsorgt. Deshalb spielt eine Verschmutzung durch aus der Einstichwunde austretendes Blut keine Rolle.

Da die Mündung des Probenaufnahmekanals, durch die die Probenaufnahme erfolgt, räumlich getrennt von der Mündung des Führungskanals ist, muß nach dem Einstich die Probenaufnahmeeinheit so bewegt werden, daß die Mündung des Probenaufnahmekanals für eine Probenaufnahme zu der Einstichwunde gebracht wird. Dazu dient der Referenzelement-Kopplungsmechanismus, der zusätzlich zu dem Lanzetten-Kopplungsmechanismus vorhanden ist. Zwar ist es prinzipiell auch möglich, die Mündung des Lanzetten-Führungskanals auch für den Probenaufnahmekanal zu nutzen, so daß die Kanäle nicht räumlich getrennt sind und die erläuterte Bewegung nicht notwendig ist. Im Rahmen der Erfindung wurde jedoch festgestellt, daß es günstiger ist, getrennte Kanäle vorzusehen und, beispielsweise durch eine Schwenkbewegung, die Mündung des Probenaufnahmekanals nach dem Einstich zu der Einstichwunde zu bringen.

Ein alternatives Ausführungsbeispiel betrifft Fälle, bei denen das Referenzelement ein mehrfach verwendbares Bauteil der Lanzettenvorrichtung ist. In diesem Fall ist eine Verschmutzung des Referenzelementes durch aus der Einstichwunde austretendes Blut selbstverständlich unerwünscht. Es wird deshalb gemäß einer bevorzugten Ausgestaltung der Erfindung nach dem Einstich rasch von der Einstichwunde wegbewegt, bevor es durch austretendes Blut verschmutzt wird.

Im Rahmen der Erfindung wurde festgestellt, daß der Anpreßdruck, mit dem das Referenzelement bei einem Einstich gegen die Haut gepreßt wird, dem Austreten von Blut aus der Einstichwunde entgegengewirkt. Diese Erkenntnis wird genutzt, indem das Referenzelement durch eine ausreichend schnelle Bewegung von der Einstichwunde entfernt wird, bevor Blut austreten und das Referenzelement (in störendem Ausmaß) verschmutzen kann.

Bevorzugt wird das Referenzelement innerhalb von 50 Millisekunden nach dem Einstich mindestens 2 mm von der Einstichwunde wegbewegt. Zum Antreiben einer derartig schnellen Rückführbewegung des Referenzelementes eignet sich insbesondere der Antriebsrotor des Lanzettenantriebs. Für einen möglichst schmerzfreien Einstich muß auch die Lanzette mit hoher Geschwindigkeit bewegt werden. Bevorzugt setzt der Referenzelement-Kopplungsmechanismus deshalb eine Drehbewegung des Antriebsrotors in eine Bewegung des Referenzelementes um, die vorzugsweise translatorisch verläuft.

Ein wichtiger Bestandteil der erfindungsgemäßen Lanzettenvorrichtung, der jedoch auch selbständige Bedeutung hat, ist eine Lanzettenantriebs-Baugruppe umfassend einen Lanzettenantrieb mit einem durch eine Antriebsfeder antreibbaren Antriebsrotor und einem Spannrotor zum Spannen der Antriebsfeder, einem ersten Lanzetten-Kopplungsmechanismus zum Umsetzen einer Drehbewegung des Antriebsrotors in eine Einstich- und Rückführbewegung der Lanzette und einen weiteren mit dem Lanzettenantrieb gekoppelten Kopplungsmechanismus.

Der weitere Kopplungsmechanismus dient bevorzugt zum Bewegen eines Referenzelementes, kann aber beispielsweise auch (zum Beispiel in einem Analysegerät mit integrierter Lanzettenvorrichtung) zum Weitertakten eines Magazins mit Teststreifen genutzt werden, zum Auftragen eines Antiseptikums auf die Einstichstelle oder zur Aufnahme einer Probe auf ein Testfeld.

Die Erfindung wird nachfolgend anhand von in den Figuren dargestellten Ausführungsbeispielen näher erläutert. Die darin dargestellten Besonderheiten können einzeln oder in Kombination verwendet werden, um bevorzugte Ausgestaltungen zu schaffen. Gleiche oder einander entsprechende Bauteile sind mit übereinstimmenden Bezugszeichen gekennzeichnet. Es zeigen:
- Fig. 1: eine perspektivische Ansicht einer Lanzettenvorrichtung;
- Fig. 2: eine perspektivische Darstellung einer Lanzettenvorrichtung mit abgenommenem Gehäuse;
- Fig. 2a: eine schematische Detail-Querschnittsdarstellung der in Fig. 2 gezeigten Lanzettenvorrichtung;
- Fig. 3: eine perspektivische Ansicht einer Lanzettenantriebs-Baugruppe mit einem Trommelmagazin;
- Fig. 4: eine weitere perspektivische Darstellung des in den Figuren 2 und 3 gezeigten Ausführungsbeispiels;
- Fig. 5: eine graphische Darstellung der Bewegung der Lanzette und des Referenzelementes bei der Baugruppe von Fig. 3 in Abhängigkeit von der Drehwinkelstellung des Antriebsrotors;
- Fig. 6: eine perspektivische Darstellung eines weiteren Ausführungsbeispiels einer LanzettenantriebsBaugruppe;
- Fig. 7: eine graphische Darstellung der Bewegung der Lanzette und des Referenzelementes bei der Baugruppe von Fig. 6 in Abhängigkeit von der Drehwinkelstellung des Spannrotors;
- Fig. 8: eine perspektivische Ansicht eines weiteren Ausführungsbeispiels einer LanzettenantriebsBaugruppe;
- Fig. 9: eine perspektivische, auseinandergezogene Darstellung der Teile des in Fig. 8 gezeigten Ausführungsbeispiels;
- Fig. 10: eine schematische Darstellung eines Referenzelementes in Form einer Probenaufnahmeeinheit; und
- Fig. 11: eine Teilansicht eines weiteren Ausführungsbeispiels einer Lanzettenantriebs-Baugruppe in perspektivischer Darstellung.

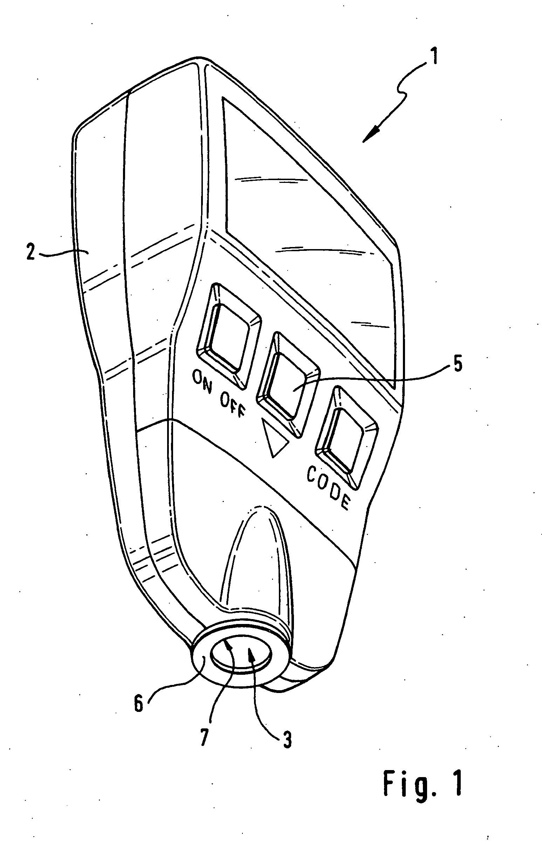

Die in Figur 1 gezeigte Lanzettenvorrichtung 1 dient zum Erzeugen einer Einstichwunde zum Entnehmen von Blut für Diagnosezwecke. Ihr Gehäuse 2 weist eine Gehäuseöffnung 3 auf. In einem vor der Gehäuseöffnung befindlichen Körperteil kann durch die Spitze einer Lanzette, die in dem Gehäuse 2 auf einem Einstichweg beweglich ist, eine Stichwunde erzeugt werden.

Die Gehäuseöffnung 3 ist von einer ringförmigen Andruckfläche 6 umgeben, mit der die Lanzettenvorrichtung für einen Einstich gegen ein Körperteil, zum Beispiel eine Fingerbeere, gedrückt wird. Dabei wölbt sich die Hautoberfläche in die Gehäuseöffnung 3 hinein. Die Vorwölbung der Haut erleichtert den Blutaustritt, so daß bereits bei einer vergleichsweise geringen Einstichtiefe von 0,5 mm bis 2 mm eine ausreichende Menge Blut für Diagnosezwecke entnommen werden kann. Durch mehrfaches Andrücken an die Haut läßt sich diese Wirkung noch zusätzlich verbessern ("Pumpwirkung"). Außerdem kann mit einem derartig gestalteten Blutentnahmegerät auch aus weniger gut durchbluteten, aber auch weniger schmerzempfindlichen, Körperteilen ein Blutstropfen entnommen werden. Um die gewünschte Pumpwirkung zu gewährleisten, sollte der Innendurchmesser der Andruckfläche 6 recht groß sein, bevorzugt mindestens 7 mm oder sogar mindestens 9 mm betragen.

Bevorzugt ist die Andruckfläche 6 nach innen zur Gehäuseöffnung 3 hin geneigt. Insbesondere ist es vorteilhaft, die Andruckfläche 6 aus einem deformierbaren Material, beispielsweise Polyurethan oder Gummi, zu bilden, wodurch sich die erwähnte Pumpwirkung beim Anpressen an ein Körperteil noch verstärken läßt. Nähere Einzelheiten und alternative Ausführungsformen der Andruckfläche 6 sind in der DE 100 26 172 A1 beschrieben, die diesbezüglich durch Bezugnahme zum Gegenstand der vorliegenden Anmeldung gemacht wird. Die Andruckfläche wird dort von einem Bauteil gebildet, das wegen seiner Pumpwirkung als Kompressionseinheit bezeichnet wird.

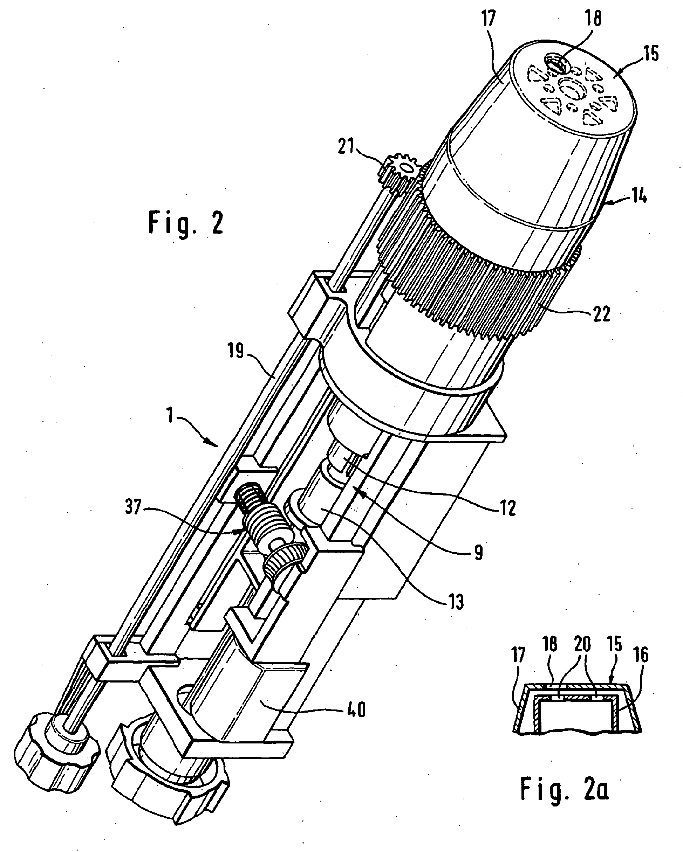

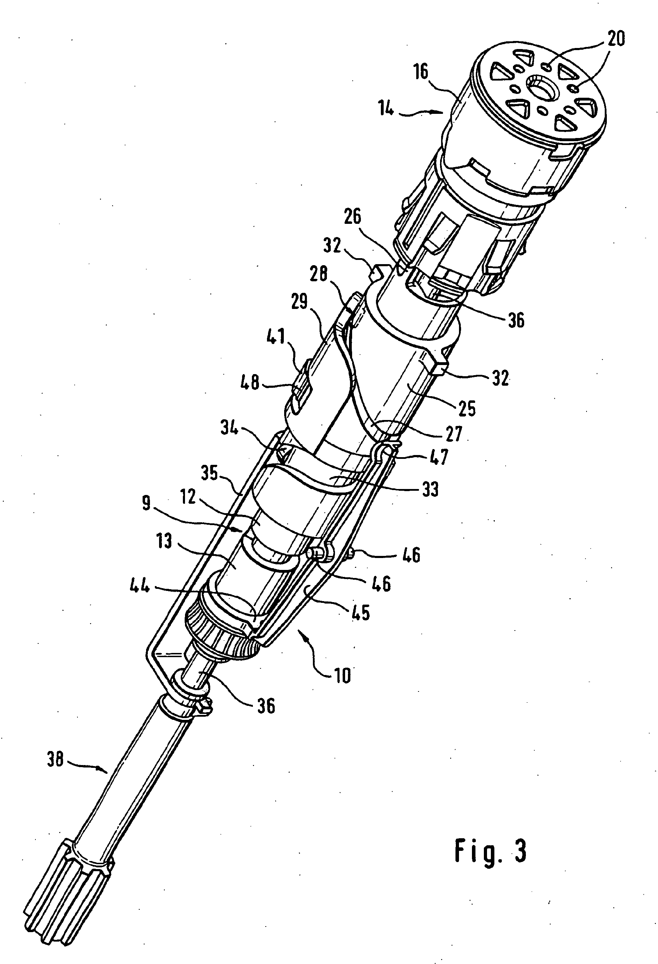

Die beschriebene Konstruktion führt dazu, daß die exakte Position der Hautoberfläche relativ zu der Lanzettenvorrichtung von dem Anpreßdruck und weiteren Faktoren (beispielsweise der Spannung der Haut) abhängt und nicht exakt definiert ist. Anhand der Figuren 2 und 3 wird im folgenden erläutert, wie dennoch gewährleistet werden kann, daß bei der Einstichbewegung der Lanzette eine Einstichwunde mit vorgegebener Einstichtiefe erzeugt wird.

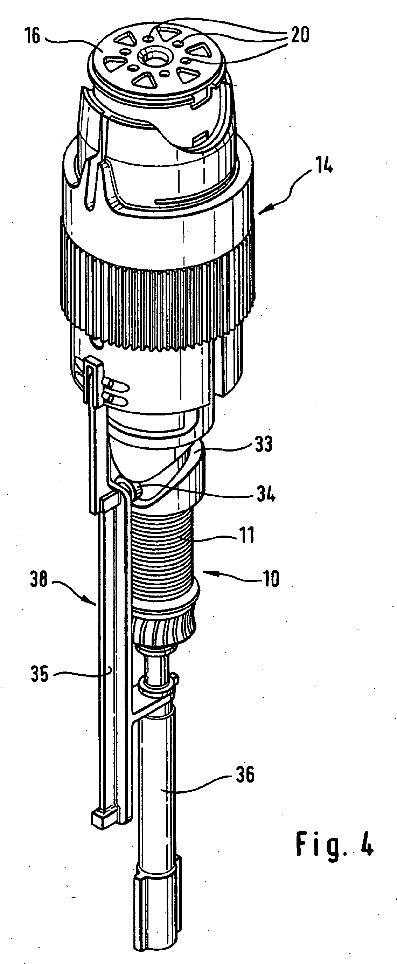

Figur 2 zeigt eine Lanzettenvorrichtung 1 bei abgenommenem Gehäuse, Figur 3 deren Lanzettenantriebs-Baugruppe 10, die mit einem Trommelmagazin 16 verbunden ist. Die Baugruppe 10 umfaßt einen Antriebsrotor 12, der durch eine Antriebsfeder antreibbar ist, und einen Spannrotor 13 zum Spannen der Antriebsfeder. Der besseren Übersichtlichkeit halber ist die Antriebsfeder, die zwischen dem Antriebsrotor 12 und dem Spannrotor 13 angeordnet ist, in den Figuren 2 und 3 nicht dargestellt. Die Antriebsfeder 11 ist lediglich in Figur 4 zu sehen. Sie ist eine Torsionsfeder, die unter Vorspannung steht und sich mit einem Ende an dem Antriebsrotor 12 und mit ihrem anderen Ende an dem Spannrotor 13 angelenkt ist.

Ein weiterer Bestandteil der Lanzettenantriebsbaugruppe 10 ist ein abtriebsseitiger Lanzetten-Kopplungsmechanismus 24, durch den die Drehbewegung des um eine Achse drehbaren Antriebsrotors 12 in einer Antriebsphase des Lanzettenantriebs in eine Einstichbewegung umgesetzt wird, bei der die Lanzette mit hoher Geschwindigkeit in Einstichrichtung bewegt wird, um eine Einstichwunde zu erzeugen. Auch die Rückführbewegung der Lanzette wird durch die Drehbewegung des Antriebsrotors 12 mittels des Lanzetten-Kopplungsmechanismus 24 angetrieben und kontrolliert. Während der gesamten Antriebsphase (welche die Einstich- und Rückführbewegung umfaßt) stützt sich das von dem Antriebsrotor 12 abgewandte Ende der Antriebsfeder gegen den Spannrotor ab, der in der Antriebsphase derart arretiert ist, daß er nicht rückwärts (gegen die Drehrichtung des Antriebsrotors) gedreht werden kann. In einer Spannphase der Lanzettenantriebsbewegung ist der Spannrotor zum Spannen der Antriebsfeder bei gehemmter Rotation des Antriebsrotors 12 in die gleiche Drehrichtung drehbar, in die sich der Antriebsrotor 12 während der Antriebsphase dreht. Ein solcher Lanzettenantrieb ist in der EP 1384438 A1 beschrieben, deren Inhalt, insbesondere hinsichtlich der Details des Lanzettenantriebs, durch Bezugnahme zum Gegenstand der vorliegenden Anmeldung gemacht wird.

Die Lanzettenantriebs-Baugruppe 10 umfaßt ferner einen weiteren Kopplungsmechanismus 38, mit dem eine Drehbewegung eines rotierenden Bauelementes des Lanzettenantriebs in eine Bewegung des Referenzelementes 14 oder eines anderen Bauteils einer Lanzettenvorrichtung umsetzbar ist. Da dieser weitere Kopplungsmechanismus 38 bei dem beschriebenen Ausführungsbeispiel zum Bewegen des Referenzelementes 14 dient, wird er in diesem Zusammenhang als Referenzelement-Kopplungmechanismus bezeichnet.

Das Referenzelement ist relativ zu der Lanzette und relativ zu dem Gehäuse beweglich. Es ist so ausgebildet und angeordnet, daß es zum Zeitpunkt des Einstichs der Lanzette mit seiner Kontaktfläche in einer definierten Position relativ zu dem Lanzettenantrieb an der Hautoberfläche eines Patienten anliegt. Dadurch wird sichergestellt, daß eine Einstichwunde mit vorgegebener Einstichtiefe erzeugt wird, die dem Abstand zwischen der Kontaktfläche des Referenzelementes und der sich am meisten in Einstichrichtung erstreckenden Position der Lanzettenspitze entspricht.

Bei dem in Figur 2 gezeigten Ausführungsbeispiel schließt das Referenzelement 14 ein Trommelmagazin 16, in dem mehrere Lanzetten gelagert werden können, und eine das Trommelmagazin 16 umgebende Schutzkappe 17 ein. Die Kontaktfläche 15 ist an der Schutzkappe 17 ausgebildet, mit der das Referenzelement 14 an der Hautoberfläche anliegt. Wie insbesondere die schematische Darstellung des vorderen Teils des Referenzelements 14 in Figur 2a zeigt, ist in der Kontaktfläche 15 eine Lanzettenöffnung 18 ausgebildet, durch welche die Lanzette bei einem Einstich austritt, um in einem gegen die Gehäuseöffnung 3 (d.h. gegen die Andruckfläche 6) gepreßten Finger eine Einstichwunde zu erzeugen. In Figur 4 ist das Referenzelement 14 bei abgenommener Schutzkappe 17 und seine Verbindung mit dem Referenzelement-Kopplungsmechanismus 38 dargestellt.

Für eine sterile Lagerung von Lanzetten bis zu ihrer Verwendung ist es bevorzugt, wenn das Trommelmagazin 16 mehrere Kammern aufweist, in denen jeweils eine einzige Lanzette gelagert wird. Möglich ist es aber auch, die Lanzetten ringförmig in einem Trommelmagazin 16 anzuordnen, das nicht in einzelne Kammern unterteilt ist. Die Anzahl der in einem Trommelmagazin 16 gelagerten Lanzetten ist weitgehend beliebig wählbar. Bevorzugt faßt ein Trommelmagazin 16 drei bis zwölf, besonders bevorzugt vier bis acht Lanzetten. Das Trommelmagazin 16 ist relativ zu der Lanzettenöffnung 18 der Schutzkappe 17 so drehbar, daß Austrittsöffnungen 20 des Trommelmagazins 16 der Reihe nach für einen Einstich fluchtend mit der Lanzettenöffnung 18 positionierbar sind.

Zu dem Lanzetten-Kopplungsmechanismus 24 gehört ein drehfest mit dem Gehäuse verbundener Schubzylinder 25, von dem eine Kopplungsstange 26 ausgeht, die während der Einstich- und Rückführbewegung mit jeweils einer ("aktiven") Lanzette verbunden ist. Der Lanzetten-Kopplungsmechanismus 24 setzt die Rotationsbewegung des Antriebsrotors 12 mittels einer Kurvensteuerung in eine Translationsbewegung der aktiven Lanzette um.

Zu diesem Zweck ist der Schubzylinder 25 mit einer Ausnehmung in Form einer Nut versehen, die eine Steuerkurve 27 bildet und von einem Steuerkurvenreiter 28 des Antriebsrotors 12 abgefahren wird. Bei dem gezeigten Ausführungsbeispiel ist der Steuerkurvenreiter 28 als Steuerzapfen ausgebildet, der in die Nut eingreift und am Ende eines von dem Antriebsrotor 12 ausgehenden Steuerarmes 29 sitzt. Der Schubzylinder 25 ist mit Führungselementen 32 versehen, die in axial verlaufende Längsnuten in der Innenwand des Gehäuses 2 (nicht dargestellt) eingreifen, um eine drehfeste, aber in Einstichrichtung bewegliche Verbindung des Schubzylinders 25 in dem Gehäuse 2 zu erreichen. Bei dem gezeigten Ausführungsbeispiel sind die Führungselemente 32 als zwei Führungszapfen verwirklicht.

Der im folgenden anhand von den Figuren 3 und 4 beschriebene Referenzelement-Kopplungsmechanismus 38, mit dem nach einem Einstich das Referenzelement 14 von der Einstichwunde zurückgezogen wird, arbeitet ebenfalls mit einer Kurvensteuerung. Ihre Steuerkurve 33 wird von einer Ausnehmung in Form einer Nut in dem Antriebsrotor 12 gebildet, in die ein Steuerkurvenreiter 34 in Form eines Steuerzapfens eingreift. Er ist über einen Kopplungsarm 35 mit einer Führungsstange 36 verbunden, an der das Trommelmagazin 16 befestigt ist. Der Kopplungsarm 35 trägt das Referenzelement 14. Die Führungsstange 36 ist gegenüber dem Kopplungsarm 35 drehbar, so daß über die Führungsstange 36 das Trommelmagazin 16 in dem Referenzelement 14 gedreht werden kann, und auf diese Weise die in dem Trommelmagazin 16 gelagerten Lanzetten der Reihe nach verwendet werden können.

Zur Einstellung der Stechtiefe wird der Abstand zwischen dem Trommelmagazin 16 und der die Kontaktfläche 15 ausbildenden Schutzkappe 17 verändert, indem die Länge des Referenzelements 14 durch ein Gewinde (nicht gezeigt) eingestellt wird. Die Einstellung erfolgt über die in Figur 2 gezeigte Welle 19, die über ein Zahnrad 21 mit einem Zahnkranz 22 des Referenzelements 14 im Eingriff steht.

Zum Spannen der Antriebsfeder wird der Spannrotor 13 über ein Getriebe 37, das bevorzugt von einem batteriegetriebenen Elektromotor 40 (Fig. 2) angetrieben wird, in Rotation versetzt. Der Spannrotor 13 kann aber auch mittels einer aus dem Gehäuse 2 herausragenden Welle von Hand gedreht werden.

Um die Federkraft der Antriebsfeder 11 möglichst effizient für eine rasche Einstich- und Rückführbewegung nutzen zu können, hat es sich als vorteilhaft erwiesen, wenn vorbereitende Bewegungen der Lanzette und des Referenzelementes 14 bereits vor dem Spannen oder spätestens während des Spannens der Antriebsfeder durchgeführt werden.

Aus diesem Grund durchlaufen der Antriebsrotor 12 und der Spannrotor 13 zunächst in einer Vorbereitungsphase einen Vorbereitungsdrehwinkelbereich, bevor die Antriebsfeder gespannt wird. Dabei wird eine Drehbewegung des Spannrotors 13 über die vorgespannte Antriebsfeder so auf den Antriebsrotor 12 übertragen, daß dieser während mindestens eines Teils der Vorbereitungsphase gemeinsam (d.h. gleichzeitig, jedoch nicht notwendigerweise, aber bevorzugt, synchron) mit dem Spannrotor 13 gedreht wird. Über einen der Kopplungsmechanismen wird die Bewegung des Antriebsrotors 12 in dem Vorbereitungsdrehwinkelbereich in eine Vorbereitungsbewegung der Kopplungsstange 26 relativ zu dem Trommelmagazin 16 umgesetzt, bei welcher die Kopplungsstange 26 durch eine einer Lanzettenöffnung 18 gegenüberliegende Einschuböffnung (nicht dargestellt) in eine Kammer des Trommelmagazins 16 eindringt und mit einer darin befindlichen Lanzette verrastet. Nähere Einzelheiten und alternative Ausführungsformen eines geeigneten Kopplungsmechanismus sind in der WO 02/36010 A1 beschrieben, die diesbezüglich durch Bezugnahme zum Gegenstand der vorliegenden Anmeldung gemacht wird.

Während der Antriebsrotor 12 und der Spannrotor 13 den Vorbereitungsdrehwinkelbereich durchlaufen, wird durch den Referenzelement-Kopplungsmechanismus 38 das Referenzelement 14 in dem Gehäuse 2 so weit in Richtung auf dessen Gehäuseöffnung 3 vorgeschoben, daß es mit der Kontaktfläche 15 an der Haut eines Patienten anliegen kann. Bedingt durch die Größe des Innendurchmessers der Andruckfläche 6 wölbt sich die Fingerbeere beim Anlegen etwas in die Gehäuseöffnung 3 des Lanzettengeräts 1 (Fig. 1) hinein. Das Referenzelement 14 kann deshalb mit seiner Kontaktfläche 15 an der Haut anliegen, obwohl es sich in dem Gehäuse 2 in einer Position befindet, bei der die Kontaktfläche 15 mit Abstand hinter einer durch den inneren Rand 7 der Andruckfläche 6 verlaufenden Ebene verläuft.

Wenn der Antriebsrotor 12 den Vorbereitungsdrehwinkelbereich durchlaufen hat, wird er mittels eines Sperrelementes 41 arretiert, so daß während der anschließenden Spannphase der Lanzettenantriebsbewegung bei weiterer Drehung des Antriebsrotors 13 die Antriebsfeder gespannt wird. Bei dem gezeigten Ausführungsbeispiel ist das Sperrelement 41 als federnd an einer Innenwand des Gehäuses 2 anliegende Auslösezunge ausgebildet, die von dem Antriebsrotor 12 ausgeht und zum Arretieren des Antriebsrotors 12 in eine Ausnehmung in der Innenwand des Gehäuses eingreift. Die Auslösezunge ist einstückig mit dem Steuerarm 42 und dem Steuerzapfen 28 als Spritzgußteil hergestellt.

Ist der Antriebsrotor 12 durch das Sperrelement 41 arretiert, so führt eine weitere Drehbewegung des Spannrotors 13 dazu, daß die Antriebsfeder gespannt wird. An dem Spannrotor 13 befinden sich elektrische Kontakte (nicht dargestellt) zum Abschalten des Elektromotors 40, wenn der Spannrotor 13 einen vorgegebenen Spanndrehwinkelbereich durchlaufen hat. Dadurch wird die Spannphase beendet. Wird der Spannrotor 13 von Hand angetrieben, so kann er nach Durchlaufen des Spanndrehwinkelbereichs durch eine mechanische Verrastung blokkiert werden.

Eine Einstichbewegung wird nach abgeschlossenem Spannvorgang dadurch eingeleitet, daß das Sperrelement 41 aus seinem Blockadeeingriff gelöst wird, so daß sich in der Antriebsphase der Antriebsrotor 12, getrieben von der Antriebsfeder, sehr schnell in seine Ruheposition dreht. Er dreht sich dabei in dieselbe Drehrichtung wie der Spannrotor 13 beim Spannen der Antriebsfeder. Diese schnelle Bewegung des Antriebsrotors 12 wird über den Lanzetten-Kopplungsmechanismus 24 in eine Einstich- und Rückführbewegung der Lanzette und über den Referenzelement-Kopplungsmechanismus 38 in eine Rückführbewegung des Referenzelementes 14 umgesetzt.

Bei der Rückführbewegung des Referenzelementes 14 handelt es sich ebenso wie bei der Rückführbewegung der Lanzette um eine Translationsbewegung in axialer Richtung. Dadurch, daß die rasche Bewegung des Antriebsrotors 12 sowohl für die Rückführbewegung des Referenzelementes 14 als auch für die Rückführbewegung der Lanzette genutzt wird, kann man erreichen, daß das Referenzelement 14 so rasch von der Einstichwunde entfernt wird, daß es praktisch nicht von austretendem Blut verschmutzt wird.

Der Einstichvorgang kann beispielsweise dadurch ausgelöst (getriggert) werden, daß die Kontaktfläche 15 des Referenzelementes 14 mit einer vorgegebenen Mindestkraft gegen die Haut gedrückt wird. Bevorzugt weist das Referenzelement 14 einen Drucksensor auf, mit dem ein zum Auslösen einer Einstichbewegung erforderliches Signal erzeugbar ist, sobald auf die Kontaktfläche 15 ein Druck ausgeübt wird, der einen vorgegebenen Mindestanpreßdruck überschreitet. Der Drucksensor kann beispielsweise dadurch verwirklicht werden, daß die Kontaktfläche 15 auf einer Feder gelagert ist und ein elektrischer Kontakt erst nach Zusammendrücken der Feder geschlossen wird.

Alternativ oder zusätzlich kann ein Auslöseknopf 5 an dem Gehäuse 2 der Lanzettenvorrichtung 1 angeordnet sein (Fig. 1). Zum Auslösen des Einstichvorgangs wird bei der dargestellten Ausführungsform zunächst ein elektrisches Signal erzeugt, durch das der Elektromotor 40 in Gang gesetzt wird. Dadurch wird der Spannrotor 13 um einen kleinen Drehwinkelschritt gedreht. Wie man in Figur 3 sieht, weist der Spannrotor 13 einen Auslösenocken 44 auf, der bei Drehung des Spannrotors 13 über eine vorgegebene Drehwinkellage hinaus einen Auslösemechanismus 45 betätigt, durch den die zuvor bestehende Hemmung des Antriebsrotors 12 gelöst wird.

Bei dem gezeigten Ausführungsbeispiel ist der Auslösemechanismus 45 als Wippe ausgebildet, die um Lagerzapfen 46 schwenkbar an dem Gehäuse 2 befestigt ist. Durch den Auslösenocken 44 des Spannrotors 13 wird ein Ende der Wippe 45 angehoben und das andere Ende der Wippe 45, an dem sich ein Kopfteil 47 befindet, abgesenkt. Das Sperrelement 41 weist eine Schulter mit einer Rückführfläche 48 auf. In dieser Bewegungsphase (in Fig. 3 nicht dargestellt) befindet sich das Kopfteil 47 über dem Sperrelement 41 und drückt auf die Rückführfläche 48 der Auslösezunge 41, wodurch diese aus ihrem Eingriff mit der Ausnehmung des Gehäuses 3 gelöst wird. Sobald das Sperrelement 41 den Antriebsrotor 12 nicht mehr blockiert, entspannt sich die Antriebsfeder, so daß die Antriebsphase der Bewegung des Lanzettenantriebs abläuft.

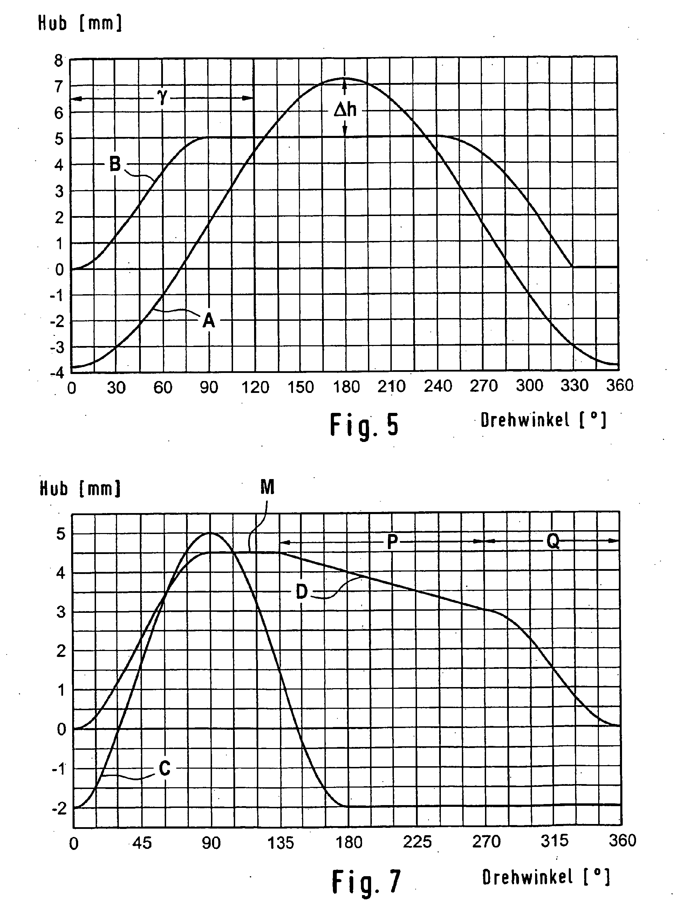

Figur 5 veranschaulicht die Bewegungen der Lanzette und des Referenzelementes 14, welche durch die Drehbewegung des Antriebsrotors 12 mittels der Kopplungsmechanismen 24, 38 angetrieben und gesteuert werden. In Figur 5 ist der Hub, also die Bewegung in axialer Richtung, der Kopplungsstange 26 als Kurve A und der Hub des Referenzelementes 14 als Kurve B über der Drehwinkelstellung des Antriebsrotors 12 aufgetragen. Dargestellt ist ein vollständiger Arbeitszyklus des Lanzettenantriebs, der eine Drehbewegung des Antriebsrotors um insgesamt 360° umfaßt. Ein Arbeitszyklus beginnt mit dem Beginn einer Drehbewegung des Spannrotors und endet mit dem Ende der Rückführbewegung der Lanzette nach erfolgtem Einstich.

Zu Beginn des Arbeitszyklus durchläuft der Antriebsrotor 12 (wie oben dargelegt in einer relativ langsamen Bewegung gleichzeitig mit dem Spannrotor) einen Vorbereitungsdrehwinkelbereich γ. Er endet bei etwa 120° (in Figur 5 durch eine senkrechte Linie gekennzeichnet). Die Bewegung des Antriebsrotors 12 in dem Vorbereitungsdrehwinkelbereich wird mittels der beiden Kopplungsmechanismen 24, 38 so umgesetzt, daß sowohl die Kopplungsstange 26 als auch das Referenzelement 14 in dem Gerät nach vorne (in Richtung zu der Gehäuseöffnung 3 hin) bewegt werden, bis die Kontaktfläche 15 des Referenzelements 14 an der Haut anliegt und die Kopplungsstange 26 an eine Lanzette angekoppelt ist.

Da die Kontaktfläche 15 zum Zeitpunkt des Einstichs an der Haut anliegt, wird die Tiefe der Einstichwunde präzise durch den Abschnitt Δh des Kopplungsstangenhubs vorgegeben, der über die Kontaktfläche 15 hinausgeht. Nach dem Einstich werden die Kopplungsstange 26 und das Referenzelement 14 in ihre Anfangspositionen zurückgezogen. Der an den Vorbereitungsdrehwinkelbereich anschließende Antriebsdrehwinkelbereich (welcher der Antriebsphase der Lanzettenantriebsbewegung, bestehend aus Einstich- und Rückführungsbewegung, entspricht) wird in weniger als 100 ms, vorzugsweise weniger als 20 ms durchlaufen.

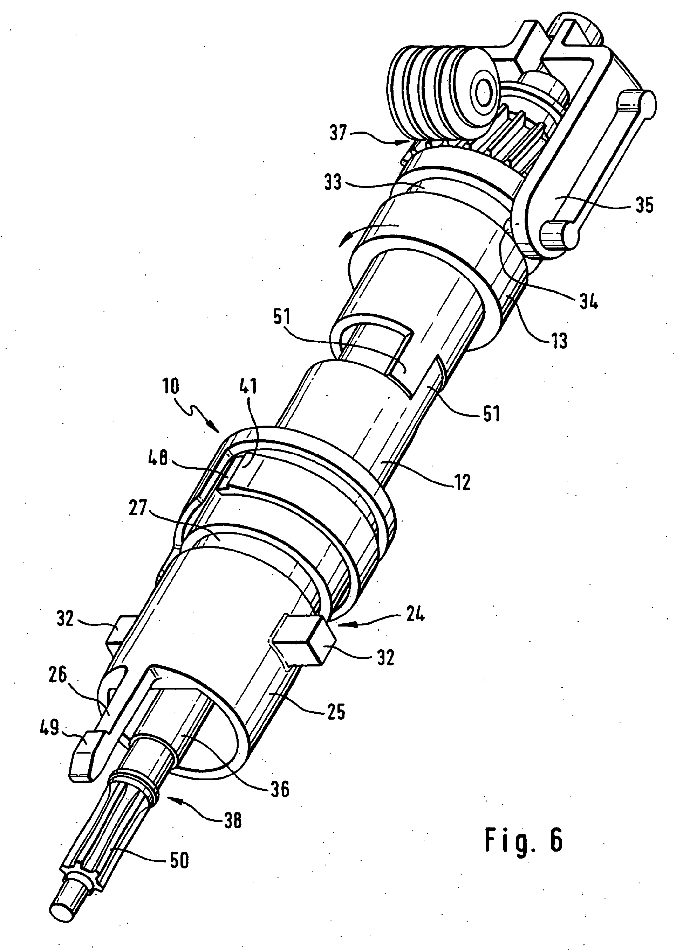

Figur 6 zeigt ein weiteres Ausführungsbeispiel einer Lanzettenantriebs-Baugruppe 10. Es ist ohne das Referenzelement 14 dargestellt, so daß der Kopf 50 der Führungsstange 36, auf den das Trommelmagazin 16 aufgesteckt wird, und die Kopplungsstange 26, die für einen Einstich mit einer Lanzette verrastet, besser zu erkennen sind. Man erkennt auch, daß der Kopplungsstangenkopf 49 eine Verdickung aufweist, die eine formschlüssige Kopplung an eine Lanzette ermöglicht. Wie bei dem anhand der Figuren 2 und 3 beschriebenen Ausführungsbeispiel ist die zwischen dem Antriebsrotor 12 und dem Spannrotor 13 angeordnete Antriebsfeder nicht dargestellt.

In Figur 6 ist zu erkennen, daß der Antriebsrotor 12 und der Spannrotor 13 Anschlagteile 51 aufweisen, die in einer Anschlagposition der Rotoren 12, 13 aneinander anschlagen und dadurch eine Drehbewegung des Antriebsrotors 12 relativ zu dem Spannrotor 13 zu stoppen. Derartige Anschlagteile 51 sind auch bei dem im vorhergehenden beschriebenen Ausführungsbeispiel vorhanden. Sie dienen insbesondere dazu, die Vorspannung der Antriebsfeder aufzunehmen und am Ende der Einstich- und Rückführbewegung den Antriebsrotor 12 zu stoppen.

Wie bereits erwähnt, wird in dem Vorbereitungsdrehwinkelbereich eine Drehbewegung des Spannrotors 13 über die Antriebsfeder 11 auf den Antriebsrotor 12 übertragen, so daß sich der Spannrotor 13 und der Antriebsrotor 12 in diesem Drehwinkelbereich gemeinsam drehen. Durch die Vorspannung der Antriebsfeder 11 wird eine enge Kopplung des Spannrotors 13 mit dem Antriebsrotor 12 erreicht, so daß die Drehwinkellage des Spannrotors 13 während der gemeinsamen Drehung von der Drehwinkellage des Antriebsrotors höchstens um 10°, vorzugsweise höchstens um 5°, abweicht. Eine ausreichend hohe Vorspannung der Antriebsfeder 11 wird auf diese Weise dazu genutzt, eine weitgehend synchrone, also winkel- und geschwindigkeitstreue Bewegung der beiden Rotoren zu erreichen. Dies hat den Vorteil, daß die Bewegungen der Kopplungsmechanismen 24, 38 noch präziser gesteuert werden können. Alternativ läßt sich eine synchrone Bewegung der beiden Rotoren 12, 13 auch erreichen, indem diese während Durchlaufen des Vorbereitungsdrehwinkelbereichs durch einen Sperriegel (nicht gezeigt) miteinander gekoppelt werden.

Auch in weiteren Einzelheiten entspricht dieses Ausführungsbeispiel weitgehend dem anhand der Figuren 2 bis 4 beschriebenen Ausführungsbeispiel. Ein Unterschied besteht insbesondere insofern, als mit dem Referenzelement-Kopplungsmechanismus 38 eine Drehbewegung des Spannrotors 13 (nicht des Antriebsrotors) in eine Bewegung des Referenzelements 14 umgesetzt wird. Deshalb ist bei dem gezeigten Ausführungsbeispiel die Steuerkurve 33 des Referenzelement-Kopplungsmechanismus 38 (ebenfalls als nutförmige Ausnehmung) in dem Spannrotor 13 ausgebildet. Der Referenzelement-Kopplungsmechanismus 38 wird bei dieser Gestaltung nicht für eine schnelle Rückführbewegung des Referenzelements 14 nach einem Einstich genutzt, sondern für eine präzise Einstellung der Einstichtiefe durch eine langsame Bewegung des Referenzelements 14. Wie Figur 5 veranschaulicht, hängt die Einstichtiefe von der Position ab, in der sich die Kontaktfläche 15 des Referenzelements 14 relativ zu der Kopplungsstange 26 befindet, wenn die Einstichbewegung stattfindet. Zum Einstellen der Einstichtiefe wird das Referenzelement 14 vor einem Einstich relativ zu der Kopplungsstange 26 in axialer Richtung verschoben. Diese Einstellbewegung erfolgt unabhängig von der Einstichbewegung und kann langsam ausgeführt werden. Vorzugsweise soll sich die eingestellte Position während der Einstichbewegung nicht ändern. Deshalb ist der Referenzelement-Kopplungsmechanismus 38 bevorzugt mit dem Spannrotor 13 gekoppelt, der sich relativ langsam dreht.

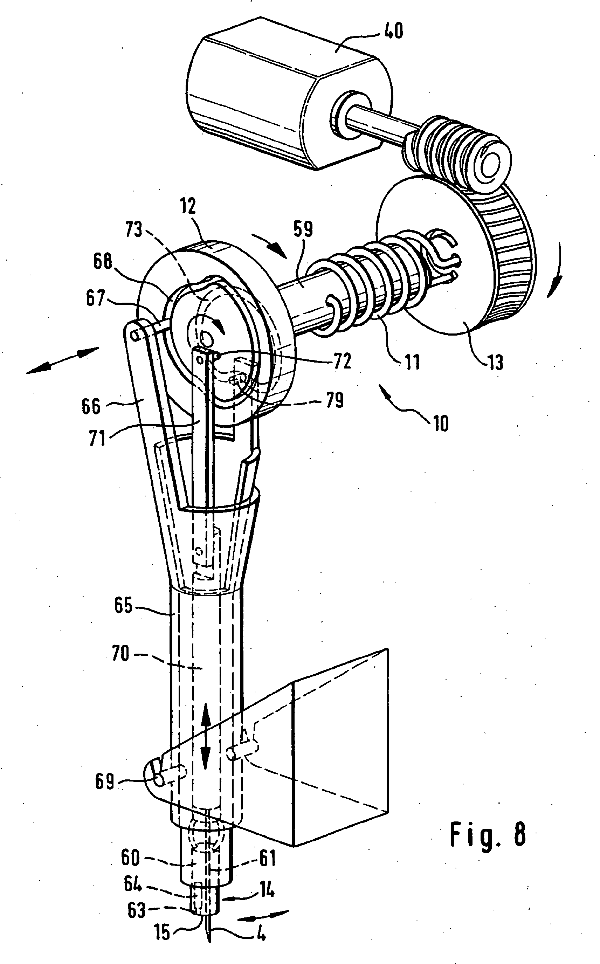

In Figur 7 ist die Bewegung der Kopplungsstange 26 und damit auch der Lanzette als Kurve C sowie die Bewegung des Referenzelementes 14 als Kurve D in Abhängigkeit von der Drehwinkelstellung des Spannrotors 13 dargestellt.

Bevorzugt erfolgt die Einstellung der Einstichtiefe nach dem Spannvorgang. Ein erster Abschnitt der Steuerkurve 33 des Referenzelement-Kopplungsmechanismus dient dazu, das Referenzelement 14 zunächst in eine Maximalposition M zu bewegen, die mit einer minimalen Einstichtiefe korrespondiert. Wird der Spannrotor 13 anschließend weitergedreht, so wird das Referenzelement 14 aus dieser Maximalposition M in eine Position zurückbewegt, die der gewünschten Einstichtiefe entspricht ("Einstichposition"). Je weiter das Referenzelement 14 relativ zu der Kopplungsstange 26 zurückgezogen wird, desto größer ist die Tiefe des anschließenden Einstichs. Ist die Einstichposition mit der gewünschten Einstichtiefe erreicht, so wird der Elektromotor 40 abgeschaltet und der Spannrotor 13 in dieser Position arretiert. Nach dem Auslösen beendet der Spannrotor 13 seinen Arbeitszyklus, indem er in dem Bewegungsabschnitt Q weitergedreht wird, bis er seine Ausgangslage erreicht.

Bei der beschriebenen Vorgehensweise zum Einstellen der Einstichtiefe ist es von großer Bedeutung, daß die Drehwinkelstellung des Spannrotors 13 exakt in der gewünschten Position arretiert wird. Eine ungenaue Positionierung der Drehwinkelstellung des Spannrotors 13 führt zu einer entsprechenden Ungenauigkeit bei der eingestellten Einstichtiefe. Bei dem gezeigten Ausführungsbeispiel treibt der Elektromotor 40 den Spannrotor 13 über ein zweistufiges Schneckengetriebe 37 an, so daß die Drehwinkelstellung des Spannrotors 13 durch eine elektronische Zählung der Umdrehungen eines oder mehrere Teile des Schneckengetriebes 37 präzise eingestellt werden kann.

Die Ausführungsbeispiele der Figuren 2 bis 4 und 6 können kombiniert werden, so daß ein erster Referenzelement-Kopplungsmechanismus das Referenzelement 14 mit dem Spannrotor 13 koppelt, um vor dem Auslösen einer Einstichbewegung die Einstichtiefe einzustellen, und ein zweiter Referenzelement-Kopplungsmechanismus das Referenzelement 14 mit dem Antriebsrotor 12 koppelt, um nach einem Einstich das Referenzelement 14 zu bewegen. Auf diese Weise wird die Antriebsfeder für eine schnelle Rückführbewegung genutzt, die sich durch einen Elektromotor 40 nur sehr schwer verwirklichen ließe, während der Elektromotor 40 für eine langsame Einstellbewegung der Einstichtiefe genutzt wird, wofür die Antriebsfeder weniger geeignet ist.

Möglich ist es auch, das Referenzelement 14 mit dem Spannrotor 13 zu koppeln, um es zur Vorbereitung einer Einstichbewegung in eine gewünschte Position zu bringen und für die Rückführbewegung des Referenzelements 14 eine separate Rückführfeder vorzusehen, die beim Vorschieben des Referenzelements 14 durch den Spannrotor 13 gespannt wird. Die Kraft der gespannten Rückführfeder kann beispielsweise dadurch aufgenommen werden, daß das Referenzelement 14 in der gewünschten Position an dem Gehäuse eingeklinkt wird. Das Zurückziehen der Hautreferenz nach einem Einstich, d.h. das Ausklinken, kann dabei vom dem Antriebsrotor 12, bevorzugt von dem Spannrotor 13, ausgelöst werden.

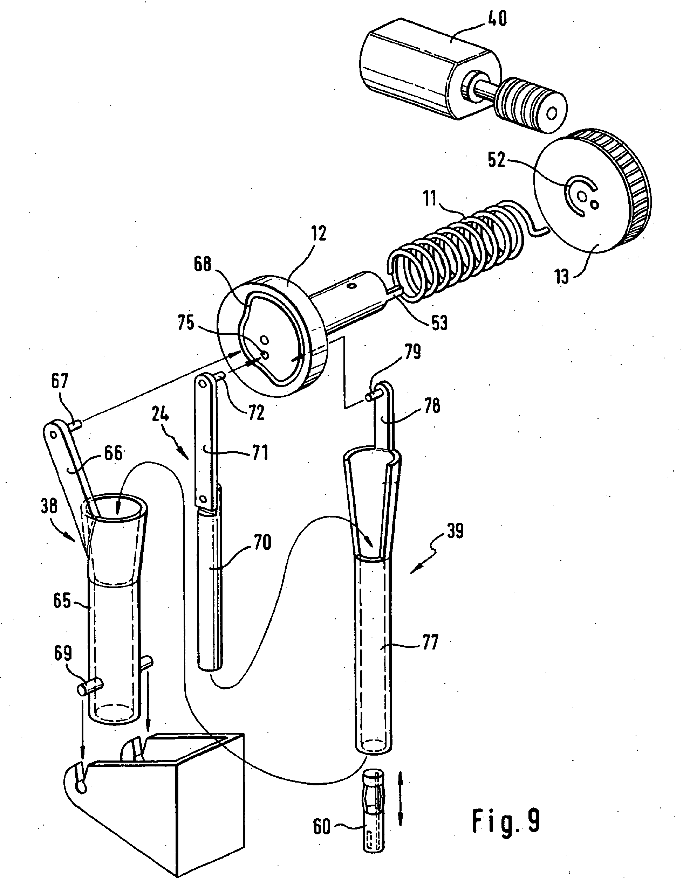

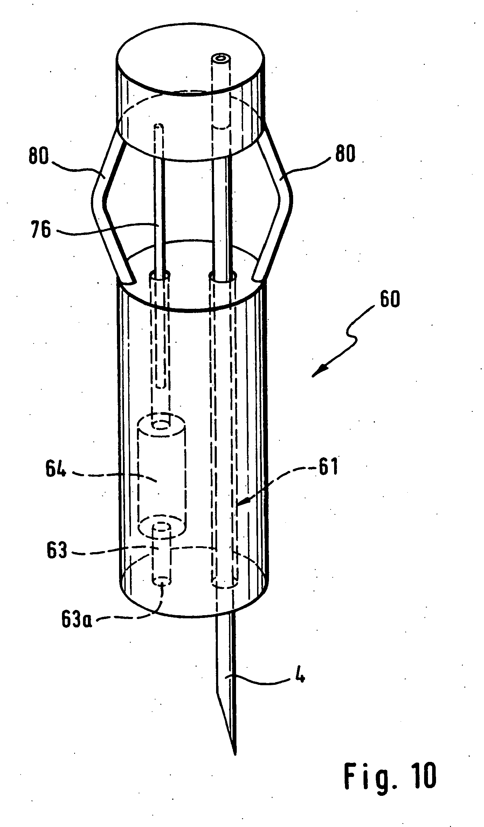

Die Figuren 8 und 9 zeigen ein weiteres Ausführungsbeispiel einer Lanzettenantriebs-Baugruppe 10. Im Unterschied zu den bisher beschriebenen Ausführungsbeispielen ist bei diesem Ausführungsbeispiel das Referenzelement 14 als Probenaufnahmeeinheit 60 ausgebildet. Wie in Figur 10 deutlicher zu erkennen ist, enthält die Probenaufnahmeeinheit 60 einen Führungskanal 61 für die Lanzette 4 und einen Probenaufnahmekanal 63 mit einer Mündung 63a zum Aufnehmen einer Probe. Die Probenaufnahmeeinheit 60 weist ferner eine Reaktionszone 64 mit Reagenzien auf, der die Probe durch den Probenaufnahmekanal 63 zuführbar ist.

Wie bei den anderen Ausführungsbeispielen umfaßt die Lanzettenantriebs-Baugruppe 10 einen durch eine Antriebsfeder 11 antreibbaren Antriebsrotor 12, der um eine Achse 59 drehbar ist, und einen Spannrotor 13 zum Spannen der Antriebsfeder 11. Antriebsrotor 12 und Spannrotor 13 sind koaxial angeordnet. Bei dem in den Figuren 8 und 9 dargestellten Ausführungsbeispiel ist die Antriebsfeder 11 eine als Schraubenfeder ausgeführte Schenkelfeder. Alternativ ist beispielsweise auch eine Spiralfeder als Antriebsfeder 11 möglich.

Die Windungen der Antriebsfeder 11 können auf Abstand gewickelt sein, um Reibungsverluste zu minimieren, oder eng aneinander anliegen, um Drehschwingungen zu dämpfen, wobei eine Vorspannung in Zugrichtung für eine dämpfende Reibung zwischen den Windungen sorgt.

Um die Vorspannung der Antriebsfeder 11 aufzunehmen, weist der Spannrotor 13 ein Anschlagteil in Form einer Anschlagnut 52 auf, in die ein Anschlagzapfen 53 des Antriebsrotors 12 eingreift. Der Anschlagzapfen 53 wird von der Vorspannung der Antriebsfeder 11 gegen ein Ende der kreisbogenförmigen Anschlagnut 52 gedrückt, an das er in einer Ruheposition anschlägt.

Nach einem Einstich wird eine Mündung 63a (Fig. 10) des Probenaufnahmekanals 63 zu der erzeugten Einstichwunde gebracht, um die Aufnahme der aus der Wunde austretenden Probenflüssigkeit zu ermöglichen. Zu diesem Zweck umfaßt der Referenzelement-Kopplungsmechanismus 38 eine um zwei Lagerzapfen 69 schwenkbar aufgehängte Hülse 65 mit einem starren Steuerarm 66, der mit einem Steuerzapfen als Steuerkurvenreiter 67 in eine durch eine nutförmige Ausnehmung gebildete Steuerkurve 68 des Antriebsrotors 12 eingreift. Die Steuerkurve 68 hat zwei halbkreisförmige Abschnitte mit unterschiedlichen Radien. Bewegt sich der Steuerkurvenreiter 67 zwischen diesen beiden Abschnitten, so bewirkt dies eine Schwenkbewegung der Hülse 65. In der Hülse 65 ist die Kopplungsstange 70 des Lanzetten-Kopplungsmechanismus 24 translatorisch beweglich, um die Einstich- und Rückführbewegung der Lanzette 4 zu ermöglichen. Die Kopplungsstange 70 wird von dem Antriebsrotor 12 über eine Pleuelstange 71 angetrieben. Die Pleuelstange 71 trägt einen Kurbelzapfen 72, der in eine Bohrung 75 des Antriebsrotors 12 eingreift.

Damit aus der Einstichwunde austretendes Blut möglichst vollständig von dem Probenaufnahmekanal 63 der Probenaufnahmeeinheit 60 aufgenommen wird, ist bei dem gezeigten Ausführungsbeispiel ein zweiter Referenzelement-Kopplungsmechanismus 39 vorgesehen, der nach der Einstichbewegung die Probenaufnahmeeinheit 60 etwas zurückzieht und nach der Schwenkbewegung wieder so nach vorne schiebt, daß ein Blutstropfen von dem Probenaufnahmekanal 63 aufgenommen werden kann.

Der zweite Referenzelement-Kopplungsmechanismus 39 umfaßt eine Hülse 77, von der ein starrer Steuerarm 78 mit einem Steuerzapfen als Steuerkurvenreiter 79 ausgeht, der in eine durch eine nutförmige Ausnehmung gebildete Steuerkurve 73 des Antriebsrotors 12 eingreift. Bei dem gezeigten Ausführungsbeispiel ist die Steuerkurve 68 auf einer ersten Seite (Vorderseite) des Antriebsrotors 12 und die Steuerkurve 73 auf seiner Rückseite angeordnet. Die Hülse 77 ist um die Kopplungsstange 70 herum und in der Hülse 65 des ersten Referenzelement-Kopplungmechanismus 38 angeordnet.

Ein vollständiger Arbeitszyklus, der in den Figuren 8 und 9 dargestellten Lanzettenvorrichtung läuft folgendermaßen ab:
Die Lanzettenvorrichtung wird an ein Körperteil eines Patienten angesetzt, so daß die Kontaktfläche 15 des von der Probenaufnahmeeinheit 16 gebildeten Referenzelements 14 an der Hautoberfläche anliegt. Zum Spannen der Antriebsfeder 11 wird der Spannrotor 13 um 180° gedreht, während der Antriebsrotor 12 von einem Arretierelement (nicht gezeigt) festgehalten wird. Zum Auslösen des Einstichs wird das Arretierelement (nicht gezeigt) gelöst, so daß der Antriebsrotor 12 eine Schnappbewegung von 180° ausführt, bis der Anschlagzapfen 53 gegen das Ende der Anschlagnut 52 des Spannrotors 53 stößt.

Bei dieser Bewegung des Antriebsrotors 12 beschreibt der Kurbelzapfen 72 die untere Hälfte einer Kreisbahn, so daß mittels der Pleuelstange 71 und der Kopplungsstange 70 des Lanzetten-Kopplungsmechanismus 24 die Einstichbewegung der Lanzette 4 ausgeführt wird. Auf dem letzten Abschnitt der Schnappbewegung des Antriebsrotors 12 bewirkt der zweite Referenzelement-Kopplungsmechanismus 39 über die Steuerkurve 73, daß die Hülse 77 und die darin gehaltene Probenaufnahmeeinheit 60 zurückgezogen wird. Da nun die Kontaktfläche 15 nicht mehr auf der Hautoberfläche anliegt, kann Probenflüssigkeit austreten.

Durch den Motor 40 wird anschließend der Spannrotor 13 nochmals um 180° bewegt, wobei der Antriebsrotor 12 wegen der Vorspannung der Antriebsfeder 11 dieser 180°-Bewegung folgt. Durch die Form der Steuerkurve 68 kommt es dabei zu einer Schwenkbewegung der Hülse 65, die so bemessen ist, daß die Mündung 63a (Figur 10) der Probenaufnahmeeinheit 60 auf die Einstichstelle ausgerichtet wird. Die Nut 73 bewirkt dabei, daß über die Pleuelstange 71 und die Kopplungsstange 70 die Probenaufnahmeeinheit 60 wieder nach vorn geschoben wird, so daß die Mündung 63a mit ausgetretenem Blut in Kontakt kommt und dieses aufsaugt.

Anstelle des Kurbelzapfens 72 und der Pleuelstange 71 kann auch eine dritte Nut in dem Antriebsrotor verwendet werden, um über einen dritten Steuerkurvenreiter die Kopplungsstange 70 anzutreiben.

Zur Verbesserung des Blutaustritts kann zusätzlich mit einem Druckstück, beispielsweise dem unteren Rand der Hülse 77, auf die Hautoberfläche in der Umgebung der Einstichstelle eingewirkt werden. Durch Anpressen eines solchen Druckstücks läßt sich Blut aus der Einstichwunde herauspressen, das von der Probenaufnahmeeinheit 60 aufgenommen werden kann. Wird nach abgeschlossener Blutentnahme die Anpreßkraft erniedrigt, so schließt sich die Einstichwunde. Nähere Einzelheiten zur Unterstützung der Blutentnahme durch ein Druckstück sind in der europäischen Patentanmeldung EP 04008691.0 offenbart, die diesbezüglich durch Bezugnahme zum Gegenstand der vorliegenden Anmeldung gemacht wird.

Die Probenaufnahmeeinheit 60 ist zusätzlich mit einem Pumpenstößel 76 (Fig. 10) versehen, um Kapillarkräfte des Probenaufnahmekanals 63 durch Ansaugkräfte zu verstärken. Die Probenaufnahmeeinheit 60 ist mit Federbeinen 80 versehen, die sich nach erfolgtem Einstich entspannen und den Pumpenstößel 76 zurückziehen, so daß Blut in den Probenaufnahmekanal 63 gesaugt wird.

Bei geeigneter Ausbildung der Steuerkurven 68 und 73 bzw. der Kopplungsmechanismen 38, 39 läßt sich zur Unterstützung der Blutentnahme auch eine pumpende Bewegung realisieren, beispielsweise indem Spannrotor 13 und Antriebsrotor 12 über einen kleinen Winkelbereich vorwärts und rückwärts bewegt werden. Ist die Vorspannung der Antriebsfeder 11 so groß, daß sich der Spannrotor 12 und der Antriebsrotor 13 im wesentlichen synchron bewegen, insbesondere deren Drehwinkellage voneinander höchstens um 10° abweicht, sind neben Vorwärtsbewegungen auch Rückwärtsbewegungen bei präziser Steuerung der Bewegungsabläufe möglich.

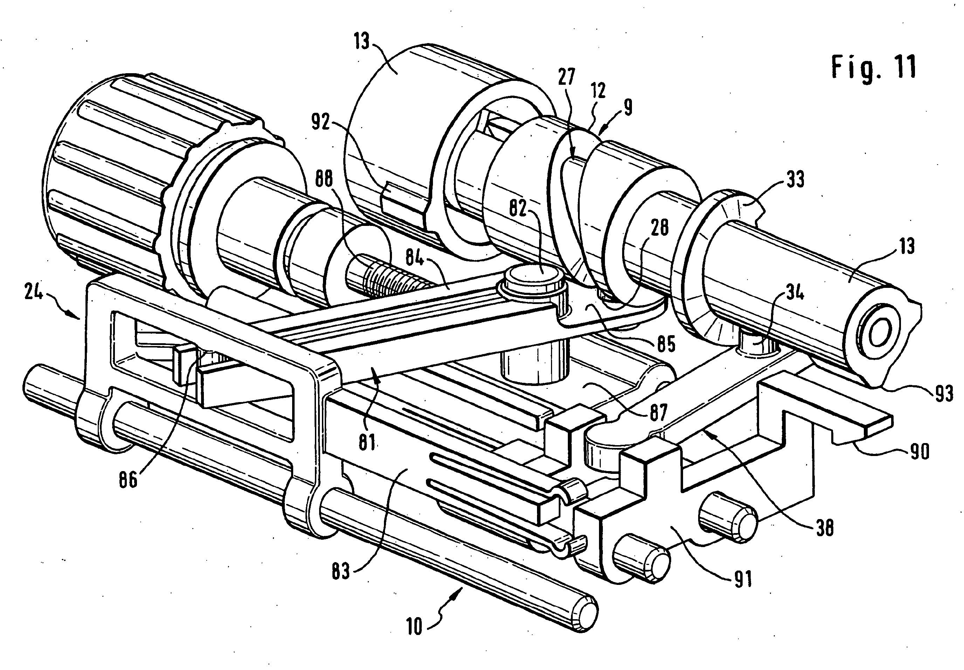

Figur 11 zeigt in einer perspektivischen Darstellung eine Teilansicht eines weiteren Ausführungsbeispiels einer Lanzettenantriebs-Baugruppe 10 mit einem Antriebsrotor 12 und einem Spannrotor 13. Zur Vereinfachung ist in Figur 11 die Antriebsfeder nicht dargestellt. Die Besonderheit der dargestellten Baugruppe 10 besteht in einem Lanzetten-Kopplungsmechanismus 24, der einen Hebel 81 aufweist, auf dem der Steuerkurvenreiter 28 zum Abfahren der als Nut ausgebildeten Steuerkurve 27 des Antriebsrotors 12 angeordnet ist. Der Hebel 81 ist drehbar in einem Drehpunkt 82 gelagert, so daß der bei Abfahren einer Flanke der Steuerkurve 27 mit dem Lanzettenhalter 83 erzielte Hub durch eine Hebelübersetzung vergrößert ist.

Die Abschnitte 84,85 des Hebels 81 zu beiden Seiten des Drehpunkts 82 bilden jeweils einen Hebelarm, so daß sich die erzielte Hubvergrößerung aus dem Verhältnis der Längen der beiden Hebelarme 84,85 berechnet. Diese Längen sind jeweils von dem Drehpunkt 82 an nach außen zu messen, d.h. bis zu dem Steuerkurvenreiter 28 bzw. dem Befestigungspunkt 86 des Lanzettenhalters 83, der von dem Hebel 81 bewegt wird.

Mit der beschriebenen Hebelübersetzung läßt sich trotz Verwendung eines kleinen Rotors 12 ein großer Hub erzielen und somit ein kleineres und kompakteres Handgerät verwirklichen. Nach dem Stand der Technik läßt sich ein großer Hub nur durch einen relativ großen Durchmesser der entsprechenden Steuerkurve 27 erzielen, da es andernfalls zu einer Selbsthemmung kommt. Überschreitet nämlich der Steigungswinkel einer Steuerkurve 27 einen kritischen Wert, der von dem Reibungskoeffizienten abhängt, blokkiert der Steuerkurvenreiter 28 und kann auch durch ein beliebig großes Drehmoment nicht in Bewegung versetzt werden. Mit der beschriebenen Hebelübersetzung läßt sich auch bei weniger steil ansteigenden Flanken einer Steuerkurve 27 ein großer Hub erzielen.

Bei dem dargestellten Ausführungsbeispiel ist die Hebelübersetzung Teil des Lanzetten-Kopplungsmechanismus 24, so daß durch Verschieben des Drehpunktes 82 die Stichtiefe eingestellt werden kann. Die Vorteile der Hebelübersetzung lassen sich jedoch auch für einen anderen Kopplungsmechanismus nutzen, der an den Antriebsrotor oder den Spannrotor gekoppelt ist.

Bei dem dargestellten Ausführungsbeispiel ist der Drehpunkt 82 als Zapfen auf einer verschieblichen Basis 87 ausgebildet. Die Basis 87 läßt sich mittels einer Gewindespindel 88 zum Einstellen der Stechtiefe verschieben. Anstelle der Gewindespindel 88 kann zum Einstellen der Lage des Drehpunktes 82 beispielsweise auch ein Exzenter oder ein Keil verwendet werden.

Bei dem dargestellten Ausführungsbeispiel ist der Spannrotor 13 durch den Antriebsrotor 12 hindurchgeführt. Der Spannrotor 13 trägt eine Steuerkurve 33, die von einem Steuerkurvenreiter 34 des Referenzelement-Kopplungsmechanismus 38 abgefahren wird. Wird der Spannrotor 13 zum Spannen der nicht dargestellten Antriebsfeder gedreht, so wird von dem Referenzelement-Kopplungsmechanismus 38 ein Referenzelement-Träger 91 vorgeschoben. Der Referenzelement-Träger 91 hat einen Rastmechanismus 90 in Form eines Rasthakens, der dabei mit dem nicht dargestellten Gehäuse verrastet. Auf diese Weise wird das Referenzelement für einen Einstich gegenüber dem Lanzettenantrieb 9 und gegenüber dem nicht dargestellten Gehäuse fixiert.

Da das Referenzelement bei einem Einstich an die Haut angepreßt wird, lasten auf ihm Andruckkräfte, die auf diese Weise über den Rastmechanismus 90 von dem Gehäuse aufgenommen werden können. Dies hat den Vorteil, daß die Kurvensteuerungen 27, 33 des Lanzettenantriebs 9 nicht durch Andruckkräfte belastet werden und folglich Reibungskräfte minimiert werden können.

Wird der Spannrotor 13 anschließend noch weiter gedreht, wird durch einen an den Spannrotor 13 angebrachten Auslösenocken 92 ein Einstich ausgelöst. Der dazu gehörende Auslösemechanismus kann gemäß dem anhand der Figuren 1 bis 5 erläuterten Ausführungsbeispiel ausgebildet sein und ist deshalb zur Vereinfachung in Figur 11 nicht dargestellt.

Der Spannrotor 13 trägt einen weiteren Auslösenocken 93, mit dem durch einen geeigneten Mechanismus (nicht dargestellt) der den Rastmechanismus 90 bildende Rasthaken aus seiner Verrastung gelöst wird, so daß der Referenzelement-Träger 91 und damit auch das Referenzelement nach einem Einstich zurückgezogen werden können.

### Bezugszeichenliste

- 1: Lanzettenvorrichtung
- 2: Gehäuse
- 3: Gehäuseöffnung
- 4: Lanzette
- 5: Auslöseknopf
- 6: Andruckfläche
- 7: Rand
- 8: Fingeraufnahme
- 10: Lanzettenantriebs-Baugruppe
- 11: Antriebsfeder
- 12: Antriebsrotor
- 13: Spannrotor
- 14: Referenzelement
- 15: Kontaktfläche
- 16: Trommelmagazin
- 17: Schutzkappe
- 18: Lanzettenöffnung
- 19: Welle
- 20: Austrittsöffnung
- 21: Zahnrad
- 22: Zahnkranz
- 24: Lanzetten-Kopplungsmechanismus
- 25: Schubzylinder
- 26: Kopplungsstange
- 27: Steuerkurve
- 28: Steuerkurvenreiter
- 29: Steuerarm
- 32: Führungselemente
- 33: Steuerkurve
- 34: Steuerkurvenreiter
- 35: Kopplungsarm
- 36: Führungsstange
- 37: Getriebe
- 38: Referenzelement-Kopplungsmechanismus
- 39: Referenzelement-Kopplungsmechanismus
- 40: Elektromotor
- 41: Sperrelement
- 44: Auslösenocken
- 45: Auslösemechanismus
- 46: Lagerzapfen
- 47: Kopfteil
- 48: Rückführfläche
- 49: Kopplungsstangenkopf
- 50: Kopf
- 51: Anschlagteile
- 52: Anschlagnut
- 53: Anschlagzapfen
- 59: Achse
- 60: Probenaufnahmeeinheit
- 61: Führungskanal
- 63: Probenaufnahmekanal
- 63a: Mündung
- 64: Reaktionszone
- 65: Hülse
- 66: Steuerarm
- 67: Steuerkurvenreiter
- 68: Steuerkurve
- 69: Lagerzapfen
- 70: Kopplungsstange
- 71: Pleuelstange
- 72: Kurbelzapfen
- 73: Steuerkurve
- 75: Bohrung
- 76: Pumpenstößel
- 77: Hülse
- 78: Steuerarm
- 79: Steuerkurvenreiter
- 80: Federbein
- 81: Hebel
- 82: Drehpunkt
- 83: Lanzettenhalter
- 84,85: Hebelarme
- 86: Befestigungspunkt des Lanzettenhalters
- 87: verschiebliche Basis
- 88: Gewindespindel
- 90: Rastmechanismus
- 91: Referenzelement-Träger
- 92: Auslösenocken
- 93: Nocken

## Patentansprüche

1. Lanzettenvorrichtung zum Erzeugen einer Einstichwunde in einer Hautoberfläche, insbesondere zum Entnehmen einer Körperflüssigkeit für Diagnosezwecke, umfassend:
ein Gehäuse (2) in dem eine Lanzette (4) auf einem Einstichweg beweglich ist,
einen Lanzettenantrieb (9) mit einem durch eine Antriebsfeder (11) antreibbaren Antriebsrotor (12),
einen Lanzetten-Kopplungsmechanismus (24) durch den während eines Arbeitszyklus des Lanzettenantriebs (9) eine Drehbewegung des Antriebsrotors (12) in eine Einstich- und Rückführbewegung der Lanzette (4) auf dem Einstichweg umgesetzt wird, wobei der Lanzetten-Kopplungsmechanismus einen Steuerkurvenreiter (28) einschließt, der eine Steuerkurve (27) des Lanzetten-Kopplungsmechanismus (24) abfährt, und
ein relativ zu der Lanzette (4) und relativ zu dem Gehäuse (2) bewegliches Referenzelement (14), das während der Einstichbewegung in einer definierten Position relativ zu dem Lanzettenantrieb (9) fixiert ist und mit einer Kontaktfläche (15) an der Hautoberfläche anliegt, so daß durch die Einstichbewegung eine Einstichwunde mit reproduzierbarer Einstichtiefe erzeugt wird,
**gekennzeichnet durch** einen Referenzelement-Kopplungsmechanismus (38,39), der zum Bewegen des Referenzelements (14) mit dem Lanzettenantrieb (9) gekoppelt ist und eine Steuerkurve (33,68,73) aufweist, die von einem Steuerkurvenreiter (34,67,72) abgefahren wird.

2. Lanzettenvorrichtung nach Anspruch 1, **dadurch gekennzeichnet, daß** der Lanzettenantrieb (9) einen Spannrotor (13) zum Spannen der Antriebsfeder (11) umfaßt.

3. Lanzettenvorrichtung nach Anspruch 2, **dadurch gekennzeichnet, daß** der Antriebsrotor (12) in einem Arbeitszyklus einen Gesamtdrehwinkelbereich durchläuft, der einen Vorbereitungsdrehwinkelbereich umfaßt, in dem sich der Antriebsrotor (12) gemeinsam mit dem Spannrotor (13) dreht.

4. Lanzettenvorrichtung nach Anspruch 3, **dadurch gekennzeichnet, daß** das Referenzelement (14) mittels des Referenzelement-Kopplungsmechanismus (38,39) bewegt wird, während der Antriebsrotor (12) den Vorbereitungsdrehwinkelbereich durchläuft.

5. Lanzettenvorrichtung nach Anspruch 3 oder 4, **dadurch gekennzeichnet, daß** der Lanzetten-Kopplungsmechanismus (24) eine Kopplungsstange (26) einschließt, die an die Lanzette (4) angekoppelt wird, während der Antriebsrotor (12) den Vorbereitungsdrehwinkelbereich durchläuft.

6. Lanzettenvorrichtung nach einem der Ansprüche 2 bis 5, **dadurch gekennzeichnet, daß** der Spannrotor (13) einen Auslösenocken (44) aufweist, der bei Drehung des Spannrotors (13) über eine vorgegebene Drehwinkellage hinaus einen Auslösemechanismus (45) betätigt, durch den zum Auslösen einer Einstichbewegung eine Arretierung des Antriebsrotors (13) gelöst wird.

7. Lanzettenvorrichtung nach einem der vorhergehenden Ansprüche, **dadurch gekennzeichnet, daß** das Referenzelement (14) einen Drucksensor aufweist, mit dem ein zum Auslösen einer Einstichbewegung erforderliches Signal erzeugt wird, sobald auf die Kontaktfläche (15) ein Druck einwirkt, der einen vorgegebenen Mindestanpreßdruck überschreitet.

8. Lanzettenvorrichtung nach einem der vorhergehenden Ansprüche, **dadurch gekennzeichnet, daß** mittels des Referenzelement-Kopplungsmechanismus (38,39) eine Drehbewegung des Antriebsrotors (12) in eine Bewegung des Referenzelements (14) umgesetzt wird.

9. Lanzettenvorrichtung nach einem der Ansprüche 2 bis 7, **dadurch gekennzeichnet, daß** mittels des Referenzelement-Kopplungsmechanismus (38) eine Drehbewegung des Spannrotors (13) in eine Bewegung des Referenzelements (14) umgesetzt wird.

10. Lanzettenvorrichtung nach einem der Ansprüche 2 bis 9, **dadurch gekennzeichnet, daß** ein erster Referenzelement-Kopplungsmechanismus (38) das Referenzelement (14) mit dem Spannrotor (13) koppelt, vorzugsweise um vor dem Auslösen einer Einstichbewegung eine Einstichtiefe einzustellen, und ein zweiter Referenzelement-Kopplungsmechanismus (39) das Referenzelement (14) mit dem Antriebsrotor (12) koppelt, vorzugsweise um das Referenzelement (14) nach einem Einstich von der Einstichwunde zu entfernen.

11. Lanzettenvorrichtung nach einem der vorhergehenden Ansprüche, **dadurch gekennzeichnet, daß** das Referenzelement (14) in dem Gehäuse (2) so angeordnet ist, daß seine Kontaktfläche zum Zeitpunkt des Einstichs mit Abstand hinter einer Ebene liegt, die durch den inneren Rand (7) einer Andruckfläche (6) verläuft, mit der die Lanzettenvorrichtung gegen ein Körperteil, aus dem eine Probe entnommen werden soll, gedrückt wird.

12. Lanzettenvorrichtung nach einem der vorhergehenden Ansprüche, **dadurch gekennzeichnet, daß** das Referenzelement (14) ein Magazin (16) einschließt, in dem mehrere Lanzetten (4) lagerbar sind.

13. Lanzettenvorrichtung nach einem der vorhergehenden Ansprüche, **dadurch gekennzeichnet, daß** sie eine Probenaufnahmeeinheit (60) aufweist, die nach einem Einstich zur Aufnahme einer Probe zu der Einstichstelle bewegt wird.

14. Lanzettenvorrichtung nach Anspruch 13, **dadurch gekennzeichnet, daß** das Referenzelement (14) die Probenaufnahmeeinheit (60) einschließt.

15. Lanzettenvorrichtung nach Anspruch 14, **dadurch gekennzeichnet, daß** die Probenaufnahmeeinheit (60) eine Führung (61) für die Lanzette (4) und einen Probenaufnahmekanal (63) zum Aufnehmen einer Probe aufweist.

16. Lanzettenvorrichtung nach einem der Ansprüche 13 bis 15, **dadurch gekennzeichnet, daß** das Referenzelement eine Reaktionszone (64) mit Reagenzien einschließt, die mit dem Probenaufnahmekanal (63) derart verbunden ist, daß in den Probenaufnahmekanal (63) aufgenommene Flüssigkeit zu der Reaktionszone (64) transportiert wird.

17. Lanzettenvorrichtung nach Anspruch 16, **dadurch gekennzeichnet, daß** die Probenaufnahmeeinheit (63) mittels des Referenzelement-Kopplungsmechanismus (38) derartig bewegt wird, daß eine Probenaufnahmeöffnung des Probenaufnahmekanals (63) zur Aufnahme einer Probe zu der Einstichwunde gebracht wird.

18. Lanzettenvorrichtung nach Anspruch 17, **dadurch gekennzeichnet, daß** die Bewegung der Probeaufnahmeeinheit (63) eine Schwenkbewegung einschließt.

19. Lanzettenvorrichtung nach einem der vorhergehenden Ansprüche, **dadurch gekennzeichnet, daß** das Referenzelement (14) durch einen Rastmechanismus (90) relativ zu dem Gehäuse fixierbar ist.

20. Lanzettenantriebs-Baugruppe, umfassend:
einen Lanzettenantrieb (9) mit einem durch eine Antriebsfeder (11) antreibbaren Antriebsrotor (12) und einem Spannrotor (13) zum Spannen der Antriebsfeder (11),
einen Lanzetten-Kopplungsmechanismus (24) mit einer Steuerkurve (27), die zum Umsetzen einer Drehbewegung des Antriebsrotors (12) in eine Einstich- und Rückführbewegung der Lanzette (14) von einem Steuerkurvenreiter (28) abgefahren wird, und
einen weiteren mit dem Lanzettenantrieb (9) gekoppelten Kopplungsmechanismus (38,39) mit einer weiteren Steuerkurve (33,68,73), die von einem Steuerkurvenreiter (34,67,72) abgefahren wird.

21. Lanzettenantriebs-Baugruppe nach Anspruch 20, **dadurch gekennzeichnet, daß** der weitere Kopplungsmechanismus (38,39) mit dem Spannrotor 13 gekoppelt ist.

22. Lanzettenantriebs-Baugruppe nach Anspruch 20 oder 21, **dadurch gekennzeichnet, daß** in einem Vorbereitungsdrehwinkelbereich eine Drehbewegung des Spannrotors (13) durch eine Kopplung auf den Antriebsrotor (12) übertragen wird, so daß sich der Spannrotor (13) und der Antriebsrotor (12) in dem Vorbereitungsdrehwinkelbereich gemeinsam drehen, wobei die Kopplung so beschaffen ist, daß die Drehwinkellage des Spannrotors (13) während der gemeinsamen Drehung in dem Vorbereitungsdrehwinkelbereich von der Drehwinkellage des Antriebsrotors (12) höchstens 10°, vorzugsweise höchstens 5°, abweicht.

23. Lanzettenantriebs-Baugruppe nach Anspruch 22, **dadurch gekennzeichnet, daß** die Kopplung durch einen Sperriegel erfolgt, durch den der Spannrotor (13) während des Vorbereitungsdrehwinkelbereichs starr mit dem Antriebsrotor (12) koppelbar ist.

24. Lanzettenantriebs-Baugruppe nach Anspruch 22, **dadurch gekennzeichnet, daß** die Kopplung dadurch erreicht wird, daß die Antriebsfeder (11) unter Vorspannung zwischen dem Antriebsrotor (12) und dem Spannrotor (13) angeordnet ist.

25. Lanzettenantriebs-Baugruppe nach Anspruch 24, **dadurch gekennzeichnet, daß** der Antriebsrotor (12) und der Spannrotor (13) Anschlagteile (51,52,53) aufweisen, die in einer Anschlagposition des Antriebsrotors (12) relativ zu dem Spannrotor (13) aneinander anschlagen und die Vorspannung der Antriebsfeder (11) aufnehmen.

26. Lanzettenantriebs-Baugruppe nach einem der Ansprüche 20 bis 25, **dadurch gekennzeichnet, daß** der Spannrotor (13) zum Spannen der Antriebsfeder (11) in die gleiche Drehrichtung drehbar ist, in die sich der Antriebsrotor (12) beim Entspannen der Antriebsfeder (11) dreht.

27. Lanzettenantriebs-Baugruppe nach einem der Ansprüche 20 bis 25, **dadurch gekennzeichnet, daß** zur Vergrößerung eines durch eine Steuerkurvenflanke bewirkten Hubs mindestens einer der Steuerkurvenreiter (28,34,67,72,79) auf einem Hebel (81) angeordnet ist, der drehbar in einem Drehpunkt (82) gelagert ist.

28. Lanzettenantriebs-Baugruppe nach Anspruch 27, **dadurch gekennzeichnet, daß** der Drehpunkt (82) zur Einstellung der durch den Hebel (81) erzielten Hubvergrößerung verschieblich ist.

## Claims

1. Lancet device for generating a puncture wound in a skin surface, in particular for withdrawing a body liquid for diagnostic purposes, comprising:
a housing (2), in which a lancet (4) can be moved along a puncturing path;
a lancet drive (9) with a drive rotor (12) that can be driven by a drive spring (11);
a lancet coupling mechanism (24) that transforms a rotational motion of the drive rotor (12) into a puncturing and returning motion of the lancet (4) along the puncturing path during a working cycle of the lancet drive (9), the lancet coupling mechanism comprising a steering curve traveler (28) traveling along a steering curve (27) of the lancet coupling mechanism (24); and
a reference element (14) that is mobile relative to the lancet (4) and relative to the housing (2) but at the time of puncturing is located in a defined position relative to the lancet drive (9) and rests with a contact surface (15) thereof against the skin surface such that a puncture wound with a reproducible puncturing depth is generated by the puncturing motion,
**characterized by** a reference element coupling mechanism (38,39) that is coupled to the lancet drive (9) for moving the reference element (14), the reference coupling mechanism (38,39) comprising a steering curve (33,68,73) and a steering curve traveler (34,67,72) traveling along the steering curve (33,68,73).

2. Lancet device according to claim 1, **characterized in that** the lancet drive (9) comprises a tensioning rotor (13) for tensioning the drive spring (11).

3. Lancet device according to claim 2, **characterized in that**, in a working cycle, the drive rotor (12) runs through a total range of rotation angles that comprises a preparatory range of rotation angles, in which the drive rotor (12) rotates jointly with the tensioning rotor (13).

4. Lancet device according to claim 3, **characterized in that** the reference element (14) is moved by means of the reference element coupling mechanism (38,39), while the drive rotor (12) rotates through the preparatory range of rotation angles.

5. Lancet device according to any one of claims 3 or 4, **characterized in that** the lancet coupling mechanism (24) includes a coupling rod (26) that is coupled with the lancet (4) while the drive rotor (12) rotates through the preparatory range of rotation angles.

6. Lancet device according to any one of the claims 2 to 5, **characterized in that** the tensioning rotor (13) comprises a triggering cam (44) that actuates a trigger mechanism (45) when the tensioning rotor (13) is rotated beyond a predetermined rotation angle position, whereby said trigger mechanism (45) releases a locking of the drive rotor (13) in order to start a puncturing motion.

7. Lancet device according to any one of the preceding claims, **characterized in that** the reference element (14) comprises a pressure sensor that is used to generate a signal required for starting a puncturing motion once a pressure in excess of a predetermined minimal pressure is applied to the contact surface (15).

8. Lancet device according to any one of the preceding claims, **characterized in that** by means of the reference element coupling mechanism (38,39) a rotational motion of the drive rotor (12) is transformed into a motion of the reference element (14).

9. Lancet device according to any one of claims 2 to 7, **characterized in that** by means of the reference element coupling mechanism (38) a rotational motion of the tensioning rotor (13) is transformed into a motion of the reference element (14).

10. Lancet device according to any one of claims 2 to 9, **characterized in that** a first reference element coupling mechanism (38) couples the reference element (14) to the tensioning rotor (13), preferably for setting a puncturing depth prior to the start of a puncturing motion, and a second reference element coupling mechanism (39) couples the reference element (14) to the drive rotor (12), preferably for removing the reference element (14) from the puncturing wound after a puncture.

11. Lancet device according to any one of the preceding claims, **characterized in that** the reference element (14) is arranged in the housing (2) such that at the time of puncture its contact surface is located behind a plane that extends through the inner rim (7) of a contact surface (6) that is to be used to press the lancet device against a body part from which a sample is to be withdrawn.

12. Lancet device according to any one of the preceding claims, **characterized in that** the reference element (14) includes a cartridge (16) in which a plurality of lancets (4) can be stored.

13. Lancet device according to any one of the preceding claims, **characterized in that** it comprises a sample take-up unit (60) that is moved after a puncture to the puncturing site in order to receive a sample.

14. Lancet device according to claim 13, **characterized in that** the reference element (14) comprises the sample take-up unit (60).

15. Lancet device according to claim 14, **characterized in that** the sample take-up unit (60) comprises a guide (61) for the lancet (4) and a sample reception channel (63) for receiving a sample.

16. Lancet device according to any one of the claims 13 to 15, **characterized in that** the reference element includes a reaction zone (64) with reagents, the reaction zone being connected to the sample reception channel (63) such that liquid taken up into the sample reception channel (63) is transported to the reaction zone (64).

17. Lancet device according to claim 16, **characterized in that** the sample take-up unit (63) is moved by means of the reference element coupling mechanism (38) such that a sample reception opening of the sample reception channel (63) is moved to the puncture wound in order to take up a sample.

18. Lancet device according to claim 17, **characterized in that** the motion of the sample take-up unit (63) includes a pivoting motion.

19. Lancet device according to any one of the preceding claims, **characterized in that** the reference element (14) can be fixed relative to the housing by means of a locking mechanism (90).

20. Lancet drive assembly, comprising:
a lancet drive (9) with a drive rotor (12) that can be driven by a drive spring (11) and a tensioning rotor (13) for tensioning the drive spring (11);
a lancet coupling mechanism (24) with a steering curve (27) along which travels a steering curve traveler (28) in order to transform a rotational motion of the drive rotor (12) into a puncturing and returning motion of the lancet (14); and
a further coupling mechanism (38,39) which is coupled to the lancet drive (9) and comprises a further steering curve (33,68,73) along which travels a steering curve traveler (34,67,72).

21. Lancet drive assembly according to claim 20, **characterized in that** the further coupling mechanism (38,39) is coupled to the tensioning rotor (13).

22. Lancet drive assembly according to claim 20 or 21, **characterized in that**, in a preparatory range of rotation angles, a rotational motion of the tensioning rotor (13) is transferred to the drive rotor (12) by a coupling such that the tensioning rotor (13) and the drive rotor (12) rotate jointly in the preparatory range of rotation angles, the coupling being arranged and adapted such that during the joint rotation the rotation angle position of the tensioning rotor (13) deviates from the rotation angle position of the drive rotor (12) by no more than 10°, preferably no more than 5°.

23. Lancet drive assembly according to claim 22, **characterized in that** the coupling is effected by a locking latch by means of which the tensioning rotor (13) can be coupled rigidly to the drive rotor (12) in the preparatory range of rotation angles.

24. Lancet drive assembly according to claim 22, **characterized in that** the coupling is effected by the drive spring (11) being arranged in a pretensioned state between the drive rotor (12) and the tensioning rotor (13).

25. Lancet drive assembly according to claim 24, **characterized in that** the drive rotor (12) and the tensioning rotor (13) comprise stop parts (51,52,53) that abut against each other in a stop position of the drive rotor (12) relative to the tensioning rotor (13), bearing the pre-tension of the drive spring (11).

26. Lancet drive assembly according to any one of claims 20 to 25, **characterized in that**, for tensioning of the drive spring (11), the tensioning rotor (13) is rotated in the same direction, in which the drive rotor (12) rotates during relaxation of the drive spring (11).

27. Lancet drive assembly according to any one of claims 20 to 25, **characterized in that**, in order to increase the distance of movement effected by a flank of the steering curve, at least one of the steering curve travelers (28,34,67,72,79) is arranged on a lever (81) that is pivoted on a pivot point (82).

28. Lancet drive assembly according to claim 27, **characterized in that** the pivot point (82) can be shifted in order to adjust the increase of the distance of movement effected by the lever (81).

## Revendications

1. Dispositif de lancette pour produire une piqûre dans une surface cutanée, en particulier pour prélever un fluide corporel à des fins de diagnostic, comprenant :
un boîtier (2) dans lequel une lancette (4) est mobile sur un trajet de piqûre,
un mécanisme d'entraînement de lancette (9) avec un moteur d'entraînement (12) actionnable par un ressort d'entraînement (11),
un mécanisme de couplage de lancette (24) par lequel, pendant un cycle de travail du mécanisme d'entraînement de la lancette (9), un mouvement de rotation du moteur d'entraînement (12) dans un mouvement de piqûre et de retrait de la lancette (4) est converti sur le trajet de piqûre, le mécanisme de couplage de la lancette renfermant un onglet de came (28) qui entraîne une came (27) du mécanisme de couplage de la lancette (24), et
un élément de référence (14) mobile par rapport à la lancette (4) et par rapport au boîtier (2) qui est fixé pendant le mouvement de piqûre dans une position définie par rapport au mécanisme d'entraînement de la lancette (9) et s'appuie avec une surface de contact (15) sur la surface cutanée de sorte que, par le mouvement de piqûre, une piqûre d'une profondeur reproductible soit produite,
**caractérisé par** un mécanisme de couplage de l'élément de référence (38, 39) qui est couplé pour déplacer l'élément de référence (14) au mécanisme d'entraînement de la lancette (9) et présente une came (33, 68, 73) qui est entraînée par un onglet de came (34, 67, 72).

2. Dispositif de lancette selon la revendication 1, **caractérisé en ce que** le mécanisme d'entraînement de la lancette (9) comprend un rotor de tension (13) pour tendre le ressort d'entraînement (11).

3. Dispositif de lancette selon la revendication 2, **caractérisé en ce que** le rotor d'entraînement (12) parcourt dans un cycle de travail, un angle de rotation complet qui comprend un angle de rotation préparatoire dans lequel le rotor d'entraînement (12) tourne conjointement avec le rotor de tension (13).

4. Dispositif de lancette selon la revendication 3, **caractérisé en ce que** l'élément de référence (14) se déplace au moyen du mécanisme de couplage de l'élément de référence (38, 39) pendant que le rotor d'entraînement (12) parcourt l'angle de rotation préparatoire.

5. Dispositif selon la revendication 3 ou 4, **caractérisé en ce que** le mécanisme de couplage de la lancette (24) comprend une barre de couplage (26) qui est couplée à la lancette (4) pendant que le rotor d'entraînement (12) parcourt l'angle de rotation préparatoire.

6. Dispositif de lancette selon l'une quelconque des revendications 2 à 5, **caractérisé en ce que** le rotor de tension (13) présente une came de déclenchement (44) qui actionne lors de la rotation du rotor de tension (13) un mécanisme de déclenchement (45) par une position prédéterminée de l'angle de rotation, par lequel un arrêt du rotor d'entraînement (13) pour déclencher un mouvement de piqûre est débloqué.

7. Dispositif de lancette selon l'une quelconque des revendications précédentes, **caractérisé en ce que** l'élément de référence (14) présente un capteur de pression avec lequel un signal nécessaire pour déclencher un mouvement de piqûre est produit dès qu'une pression s'exerce sur la surface de contact, qui dépasse une pression minimale.

8. Dispositif de lancette selon l'une quelconque des revendications précédentes, **caractérisé en ce qu'**un mouvement de rotation du rotor d'entraînement (12) est converti au moyen du mécanisme de couplage de l'élément de référence (38, 39) en un mouvement de l'élément de référence (14).

9. Dispositif de lancette selon l'une quelconque des revendications 2 à 7, **caractérisé en ce qu'**un mouvement de rotation du rotor de tension (13) est converti au moyen du mécanisme de couplage de l'élément de référence (38) en un mouvement de l'élément de référence (14).

10. Dispositif de lancette selon l'une quelconque des revendication 2 à 9, **caractérisé en ce qu'**un premier mécanisme de couplage de l'élément de référence (38) couple l'élément de référence (14) au rotor de tension (13), de préférence pour régler avant le déclenchement d'un mouvement de piqûre, une profondeur de piqûre et un deuxième mécanisme de couplage de l'élément de référence (39) couple l'élément de référence (14) au rotor d'entraînement (12), de préférence pour retirer l'élément de référence (14) du site de piqûre après une piqûre.

11. Dispositif de lancette selon l'une quelconque des revendications précédentes, **caractérisé en ce que** l'élément de référence (14) est disposé dans le boîtier (2) de telle sorte que sa zone de contact se situe au moment de la piqûre, loin en arrière d'un plan qui s'étend sur le bord interne (7) d'une surface de pression (6) avec laquelle le dispositif de lancette est pressé contre une partie corporelle de laquelle l'échantillon doit être prélevé.

12. Dispositif de lancette selon l'une quelconque des revendications précédentes, **caractérisé en ce que** l'élément de référence (14) comprend un magasin (16) dans lequel plusieurs lancettes (4) peuvent être stockées.

13. Dispositif de lancette selon l'une quelconque des revendications précédentes, **caractérisé en ce qu'**il présente une unité de prélèvement d'échantillon (60) qui est déplacée après piqûre pour le prélèvement d'un échantillon au site de piqûre.

14. Dispositif de lancette selon la revendication 13, **caractérisé en ce que** l'élément de référence (14) comprend l'unité de prélèvement d'échantillon (60).

15. Dispositif de lancette selon la revendication 14, **caractérisé en ce que** l'unité de prélèvement d'échantillon (60) présente un guidage (61) pour la lancette (4) et un canal de prélèvement d'échantillon (63) pour prélever un échantillon.

16. Dispositif de lancette selon l'une quelconque des revendications 13 à 15, **caractérisé en ce que** l'élément de référence comprend une zone réactionnelle (64) avec des réactifs qui sont reliés au canal de prélèvement d'échantillon (63) de telle sorte que le fluide prélevé dans le canal de prélèvement d'échantillon (63) est transporté à la zone réactionnelle (64).

17. Dispositif de lancette selon la revendication 16, **caractérisé en ce que** l'unité de prélèvement d'échantillon (63) est déplacée au moyen du mécanisme de couplage de l'élément de référence (38) de telle sorte qu'une ouverture de prélèvement d'échantillon du canal de prélèvement d'échantillon (63) est aménagée pour recevoir un échantillon du site de piqûre.

18. Dispositif de lancette selon la revendication 17, **caractérisé en ce que** le mouvement de l'unité de prélèvement d'échantillon (63) comprend un mouvement de traînée.

19. Dispositif de lancette selon l'une quelconque des revendications précédentes, **caractérisé en ce que** l'élément de référence (14) peut être fixé par un mécanisme d'arrêt (90) par rapport au boîtier.

20. Groupe d'assemblage d'entraînement de lancette, comprenant:
un dispositif d'entraînement de lancette (9) avec un moteur d'entraînement (12) pouvant être entraîné par un ressort d'entraînement (11) et un rotor de tension (13) pour tendre le ressort d'entraînement (11),
un mécanisme de couplage de lancette (24) avec une came (27) qui est entraînée, pour convertir un mouvement de rotation du rotor d'entraînement (12) en un mouvement de piqûre et de retrait de la lancette (14) d'un onglet de came (28) et
un autre mécanisme de couplage (38, 39) couplé au dispositif d'entraînement dé lancette (9), avec une autre came (33, 68, 73) qui est entraînée par un onglet de came (34, 67, 72).

21. Groupe d'assemblage d'entraînement de lancette selon la revendication 20, **caractérisé en ce que** l'autre mécanisme de couplage (38, 39) est couplé au rotor de tension 13.

22. Groupe d'assemblage d'entraînement de lancette selon la revendication 20 ou 21, **caractérisé en ce que**, dans un angle de rotation préparatoire, un mouvement de rotation du rotor de tension (13) est transmis par un couplage au moteur d'entraînement (12) de sorte que le rotor de tension (13) et le rotor d'entraînement (12) tournent ensemble dans l'angle de rotation préparatoire, le couplage étant créé de telle sorte que la position de l'angle de rotation du rotor de tension (13) pendant la rotation commune dans l'angle de rotation préparatoire s'écarte de la position de l'angle de rotation du rotor d'entraînement (12) au maximum de 10°, de préférence au maximum de 5°.

23. Groupe d'assemblage d'entraînement de lancette selon la revendication 22, **caractérisé en ce que** le couplage s'effectue par un arrêt par lequel le rotor de tension (13) peut être couplé de façon fixe avec le rotor d'entraînement (12).

24. Groupe d'assemblage d'entraînement de lancette selon la revendication 22, **caractérisé en ce que** le couplage s'effectue par le fait que le ressort d'entraînement (11) est disposé sous tension initiale entre le rotor d'entraînement (12) et le rotor de tension (13).

25. Groupe d'assemblage d'entraînement de lancette selon la revendication 24, **caractérisé en ce que** le rotor d'entraînement (12) et le rotor de tension (13) présentent des parties de butée (51, 52, 53) qui butent dans une position de butée du rotor d'entraînement (12) l'une contre l'autre par rapport au rotor de tension (13) et reçoivent la tension préalable du ressort d'entraînement (11).

26. Groupe d'assemblage d'entraînement de lancette selon l'une quelconque des revendications 20 à 25, **caractérisé en ce que** le rotor de tension (13) peut tourner pour tendre le ressort d'entraînement (11) dans le même sens de rotation que celui du rotor d'entraînement (12) lors du relâchement du ressort d'entraînement (11).

27. Groupe d'assemblage d'entraînement de lancette selon l'une quelconque des revendications 20 à 25, **caractérisé en ce que**, pour agrandir une course entraînée par un bord de la came, au moins d'un des onglets de came (28, 34, 67, 72, 79) est disposé sur un levier (81) qui est situé de façon à tourner à un point de rotation (82).

28. Groupe d'assemblage d'entraînement de lancette selon la revendication 27, **caractérisé en ce que** le point de rotation (82) peut coulisser pour ajuster l'agrandissement de la course entraînée par le levier (81).
